# EUROPEAN PATENT SPECIFICATION

(11) **EP 4 113 639 B1**
(45) Date of publication and mention of the grant of the patent: **26.02.2025**
(21) Application number: 21182520.3
(22) Date of filing: 29.06.2021
(51) Int. Cl.: H10K 85/60, H10K 71/40, H10K 50/11

(54) **ORGANIC COMPOUND OF FORMULA (I) FOR USE IN ORGANIC ELECTRONIC DEVICES, AN ORGANIC ELECTRONIC DEVICE COMPRISING A COMPOUND OF FORMULA (I) AND A DISPLAY DEVICE COMPRISING THE ORGANIC ELECTRONIC DEVICE**
ORGANISCHE VERBINDUNG VON FORMEL (I) ZUR VERWENDUNG IN ORGANISCHEN ELEKTRONISCHEN VORRICHTUNGEN, ORGANISCHE ELEKTRONISCHE VORRICHTUNG MIT EINER VERBINDUNG VON FORMEL (I) UND ANZEIGEVORRICHTUNG MIT DER ORGANISCHEN ELEKTRONISCHEN VORRICHTUNG
COMPOSÉ ORGANIQUE DE FORMULE (I) POUR UNE UTILISATION DANS DES DISPOSITIFS ÉLECTRONIQUES ORGANIQUES, DISPOSITIF ÉLECTRONIQUE ORGANIQUE COMPRENANT UN COMPOSÉ DE FORMULE (I) ET DISPOSITIF D'AFFICHAGE COMPRENANT LE DISPOSITIF ÉLECTRONIQUE ORGANIQUE

(43) Date of publication of application: 04.01.2023
(73) Proprietor: Novaled GmbH, 01099 Dresden (DE)
(72) Inventor: NÜLLEN, Max Peter, 01099 Dresden (DE); SCHULZE, Benjamin, 01099 Dresden (DE); WUDARCZYK, Jakob Jacek, 01099 Dresden (DE); ROBASCHIK, Peter, 01099 Dresden (DE); LAMPRECHT, Alexandra, 01099 Dresden (DE)
(74) Representative: Michalski Hüttermann & Partner Patentanwälte mbB

(56) References cited:
- EP-A1- 3 034 489

## Description

### Technical Field

The present invention relates to an organic compound of formula (I) for use in organic electronic devices, an organic electronic device comprising a compound of formula (I) and a display device comprising the organic electronic device

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

In the course of the production of OLEDs, several sublimation and thermal decomposition steps are employed. To this end it is especially important that the compounds used show low mass loss during heating in order to avoid tool contamination during the manufacturing of an organic electronic device.

There remains a need to provide such materials and/or methods for their production.

EP 3 034 489 A1 discloses a process for preparation of an electrically doped semiconducting material comprising a [3]-radialene p-dopant or for preparation of an electronic device containing a layer comprising a [3]-radialene p-dopant, the process comprising the steps (i) loading an evaporation source with the [3]-radialene p-dopant and (ii) evaporating the [3]-radialene p-dopant at an elevated temperature and at a reduced pressure.

### DISCLOSURE

An aspect of the present invention provides an organic compound of formula (I): 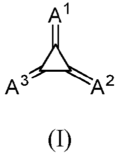
wherein A¹, A² and A³ are represented by formula (II), (III), (IV), respectively 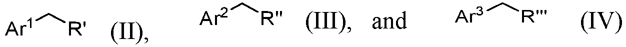
whereby Ar¹, Ar² and Ar³ are independently selected from substituted or unsubstituted aryl, substituted or unsubstituted C₆ to C₂₄ aryl, substituted or unsubstituted heteroaryl or C₂ to C₂₄ heteroaryl, wherein the substituents on Ar¹, Ar² and Ar³ are independently selected form an electron-withdrawing group, NO₂, CN, halogen, Cl, F, partially fluorinated or perfluorinated alkyl and partially fluorinated or perfluorinated C₁ to C₁₂ alkyl, CF₃, partially fluorinated or perfluorinated alkoxy, partially fluorinated or perfluorinated C₁ to C₆ alkoxy or D;
R', R'' and R‴ are independently selected from substituted or unsubstituted aryl, substituted or unsubstituted C₆ to C₁₈ aryl, substituted or unsubstituted heteroaryl, substituted or unsubstituted C₂ to C₁₈ heteroaryl, electron-withdrawing group, partially fluorinated or perfluorinated alkyl, CF₃, partially fluorinated or perfluorinated C₁ to C₆ alkyl, halogen, F, CN, and NO₂;
wherein in formulae (II) to (IV) each Ar¹, Ar² and Ar³ with the corresponding R', R'' and R‴ can form a substituted or unsubstituted carbocyle or, preferably a substituted or unsubstituted heterocycle, wherein the substituents can be electron-withdrawing group, F, CN, perfluorinated C₁ to C₈ alkyl, substituted or unsubstituted C₆ to C₁₈ aryl, substituted or unsubstituted C₂ to C₁₈ heteroaryl, substituted or unsubstituted carbocyclene, substituted or unsubstituted carbocyclidene, substituted or unsubstituted alkylene, substituted or unsubstituted alkylidene;
wherein the compound of formula (I) does not exhibit a glass transition temperature when measured by DSC during heating at 10 K/min.

In the following the term "compound of the invention" or - when that is self-explanatory - simply abbreviated "compound" shall refer to
a) A compound of formula (I) that does not exhibit a glass transition temperature when measured by DSC during heating at 10 K/min.

It should be noted that throughout the application and the claims any Aⁿ, Rⁿ etc. always refer to the same moieties, unless otherwise noted.

In the present specification, when a definition is not otherwise provided, "partially fluorinated" refers to a C₁ to C₈ alkyl group in which only part of the hydrogen atoms are replaced by fluorine atoms.

In the present specification, when a definition is not otherwise provided, "perfluorinated" refers to a C₁ to C₈ alkyl group in which all hydrogen atoms are replaced by fluorine atoms.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group, a C₁ to C₈ alkyl group, a C₁ to C₆ alkyl group, or a C₁ to C₄ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

The term "NO₂" represents - NO₂, i.e. the NO₂ is bonded by its nitrogen moiety.

The term "salt" refers to metal, ammonium, or radical cation salt of the radical anion of formula (I) preferably a radical cation salt.

The term "electron-withdrawing group" refers to a chemical group in a molecule, which can draw electrons away from an adjacent part of the molecule. The distance over which the electron-withdrawing group can exert its effect, namely the number of bonds over which the electron-withdrawing effect spans, is extended by conjugated pi-electron systems such as aromatic systems. Examples of electron-withdrawing groups include NO₂, CN, halogen, Cl, F, partially fluorinated or perfluorinated alkyl and partially fluorinated or perfluorinated C₁ to C₁₂ alkyl, partially fluorinated or perfluorinated alkoxy, partially fluorinated or perfluorinated C₁ to C₆ alkoxy.

In the present specification, the single bond refers to a direct bond.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "glass transition" especially means a gradual and reversible transition in amorphous materials (or in amorphous regions within semicrystalline materials) from a hard and relatively brittle "glassy" state into a viscous or rubbery state as the temperature is increased. Subsequently the term "glass transition temperature" especially means and/or includes the temperature and/or the range of temperatures over which this glass transition occurs.

### Advantageous Effects

Surprisingly, it was found that the compounds of the invention show lower mass loss during heating and thus undesired contamination during the production of OLEDs and other devices using these compounds can be reduced.

According to a preferred aspect of the invention the compound has been heated to ≥ 150 °C for ≥ 10 min. This heating will be in the context of this application be referred to as "thermal annealing", the temperature at which this occurs the "annealing temperature".

It should be noted that the "thermal annealing" especially occurs after the compound has been synthesized and/or purified, e.g. by sublimation etc. According to one embodiment of the invention, the "thermal annealing" is an additional treatment step, starting from a pure compound.

According to one embodiment of the present invention the compound has been heated to ≥ 155 °C, preferred ≥ 160 °C, more preferred ≥ 165 °C, yet more preferred ≥ 170 °C, still more preferred ≥ 175 °C and most preferred ≥ 180 °C.

According to one embodiment of the present invention, the compound has been heated to ≤ 300, more preferred ≤ 250 and most preferred ≤ 220 °C.

According to one embodiment of the present invention, the compound has been heated in a temperature range of ≥Tₘ-60 K to ≤ Tₘ-10 K, whereby Tₘ is the melting temperature of the compound. It has been found for many applications within the present invention that this is especially preferred when the compound is semicrystalline.

According to one embodiment of the present invention, the compound has been heated in a temperature range of ≥Tₘ-50 K to ≤ Tₘ-15 K.

According to one embodiment of the present invention, the compound has been heated in a temperature range of ≥Tₘ-40 K to ≤ Tₘ-20 K,

According to one embodiment of the present invention, the compound has been heated in a temperature range of ≥T_{aub} -60 K to ≤ T_{sub} -10 K, whereby T_{sub} is the sublimation temperature of the compound. It has been found for many applications within the present invention that this is especially preferred when the compound is amorphous and/or only shows a small degree of crystallization and/or when the compound has no defined melting temperature.

The sublimation temperature in the context of the present invention especially means and/or includes the temperature which has been measured using the following method:

### Sublimation temperature

Under nitrogen in a glovebox, 0.5 to 5 g compound are loaded into the evaporation source of a sublimation apparatus. The sublimation apparatus consist of an inner glass tube consisting of bulbs with a diameter of 3 cm which are placed inside a glass tube with a diameter of 3.5 cm. The sublimation apparatus is placed inside a tube oven (Creaphys DSU 05/2.1). The sublimation apparatus is evacuated via a membrane pump (Pfeiffer Vacuum MVP 055- 3C) and a turbo pump (Pfeiffer Vacuum THM071 YP). The pressure is measured between the sublimation apparatus and the turbo pump using a pressure gauge (Pfeiffer Vacuum PKR 251). When the pressure has been reduced to 10⁻⁵ mbar, the temperature is increased in increments of 10 to 30 K till the compound starts to be deposited in the harvesting zone of the sublimation apparatus. The temperature is further increased in increments of 10 to 30 K till a sublimation rate is achieved where the compound in the source is visibly depleted over 30 min to 1 hour and a substantial amount of compound has accumulated in the harvesting zone. The sublimation temperature, also named T_{sub1}, is the temperature inside the sublimation apparatus at which the compound is deposited in the harvesting zone at a visible rate and is measured in degree Celsius.

According to one embodiment of the present invention, the compound has been heated in a temperature range of ≥T_{aub} -50 K to ≤ T_{sub} -15 K, most preferred ≥T_{aub} -40 K to ≤ T_{sub} -20 K.

According to one embodiment of the present invention, the thermal annealing occurs for 20 min to ≤ 3hrs, more preferred ≥ 30 min to ≤ 2hrs and most preferred 45 min to ≤ 1h 30min.

According to one embodiment of the present invention, the thermal annealing occurs at reduced pressure, preferably at ≤10⁻¹ Pa, more preferably ≤10⁻² Pa, even more preferably ≤ 10⁻³ Pa and most preferred ≤ 10⁻⁴ Pa.

According to one embodiment of the present invention, the compound has a LUMO energy level, expressed in the absolute scale referring to vacuum energy level being zero of ≤ -4.5 eV calculated using the hybrid functional B3LYP with a Gaussian 6-31G* basis set as implemented in the program package TURBOMOLE V6.5. Preferably the LUMO energy level is ≤ -4.6 eV, preferably ≤ -4.7 eV, ≥ more preferably ≤-4.8 eV, and most preferably ≤ -4.9 eV.

According to one embodiment of the present invention, the compound has a melting temperature Tₘ of ≥200 °C. Preferably the melting temperature is ≥205°C and ≤450°C, more preferred ≥210°C and ≤410°C and most preferred ≥210°C and ≤380°C.

According to one embodiment of the present invention, the compound has a sublimation temperature T_{subl} of ≥165 °C, preferably ≥170 °C, more preferably ≥180 °C, even more preferably ≥190 °C, yet more preferably ≥200 °C, still more preferably ≥205 °C, and most preferably ≥210 °C. Preferably the sublimation temperature is ≥165 °C and ≤450°C, more preferred ≥170°C and ≤410°C, yet more preferred ≥180 °C and ≤ 400 °C, even more preferred ≥190 °C and ≤395 °C, more preferably ≥200 °C and ≤390 °C, still more preferably ≥205 °C and ≤385 °C and most preferred ≥210°C and ≤380°C.

According to one embodiment of the present invention, the compound has a melting enthalpy ΔHₘ of ≥ 30 kJ/mol. Preferably the melting enthalpy ΔHₘ is ≥35 kJ/mol and most preferred ≥40 kJ/mol.

According to one embodiment of the present invention, the compound compound has four to eight CN-groups, preferably five to eight CN-groups.

According to one embodiment of the present invention, the compound has a molecular mass of ≥380 g/mol. Preferably the compound has a molecular mass of ≥400 g/mol and ≤1200 g/mol, more preferred ≥500 g/mol and ≤1100 g/mol and most preferred ≥550 g/mol and ≤1000 g/mol.

According to one embodiment of the present invention, the substituents on Ar¹, Ar² and Ar³ are independently selected form NO₂, CN, halogen, Cl, F, partially fluorinated or perfluorinated alkyl and partially fluorinated or perfluorinated C₁ to C₁₂ alkyl, CF₃, partially fluorinated or perfluorinated alkoxy, partially fluorinated or perfluorinated C₁ to C₆ alkoxy or D.

According to one embodiment of the present invention, the substituents on Ar¹, Ar² and Ar³ are independently selected form NO₂, CN, F, partially fluorinated or perfluorinated alkyl and partially fluorinated or perfluorinated C₁ to C₁₂ alkyl, CF₃, or D.

According to one embodiment of the present invention, the substituents on Ar¹, Ar² and Ar³ are independently selected form NO₂, CN, F, CF₃, or D.

According to one embodiment of the present invention, R', R'' and R‴ are independently selected from electron-withdrawing group, partially fluorinated or perfluorinated alkyl, partially fluorinated or perfluorinated C₁ to C₆ alkyl, CF₃, halogen, F, CN, and NO₂.

According to one embodiment of the present invention, R', R'' and R‴ are independently selected from partially fluorinated or perfluorinated alkyl, partially fluorinated or perfluorinated C₁ to C₆ alkyl, CF₃, halogen, F, CN, and NO₂.

According to one embodiment of the present invention, R', R'' and R‴ are independently selected CF₃, F, CN, and NO₂.

According to one embodiment of the present invention, R', R'' and R‴ are independently selected CF₃, F, and CN.

According to one embodiment of the present invention, at least one of the R', R'' and R‴ is CN.

According to one embodiment of the present invention, at least one of the Ar¹, Ar² and Ar³ is selected from formula (V) 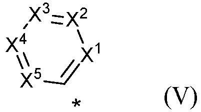
wherein X¹ is selected from CR¹ or N;
X² is selected from CR² or N;
X³ is selected from CR³ or N;
X⁴ is selected from CR⁴ or N;
X⁵ is selected from CR⁵ or N;
wherein R¹, R², R³, R⁴, and R⁵ are independently selected from electron-withdrawing group, NO₂, CN, halogen, Cl, F, partially fluorinated or perfluorinated alkyl, partially fluorinated or perfluorinated C₁ to C₁₀ alkyl, CF₃, partially fluorinated or perfluorinated alkoxy, partially fluorinated or perfluorinated C₁ to C₆ alkoxy, D or H;

wherein at least two of X¹ to X⁵ are independently selected from CR¹ to CR⁵; and
wherein the asterix "*" denotes the binding position.

According to one embodiment of the present invention, R¹, R², R³, R⁴, and R⁵ are independently selected from NO₂, CN, halogen, Cl, F, partially fluorinated or perfluorinated alkyl, partially fluorinated or perfluorinated C₁ to C₁₀ alkyl, CF₃, partially fluorinated or perfluorinated alkoxy, partially fluorinated or perfluorinated C₁ to C₆ alkoxy, D or H.

According to one embodiment of the present invention, R¹, R², R³, R⁴, and R⁵ are independently selected from NO₂, CN, F, partially fluorinated or perfluorinated alkyl, partially fluorinated or perfluorinated C₁ to C₁₀ alkyl, CF₃, partially fluorinated or perfluorinated alkoxy, partially fluorinated or perfluorinated C₁ to C₆ alkoxy, D or H.

According to one embodiment of the present invention, R¹, R², R³, R⁴, and R⁵ are independently selected from NO₂, CN, F, partially fluorinated or perfluorinated alkyl, partially fluorinated or perfluorinated C₁ to C₁₀ alkyl, CF₃, D or H.

According to one embodiment of the present invention, R¹, R², R³, R⁴, and R⁵ are independently selected from NO₂, CN, F, CF₃, D or H.

According to one embodiment of the present invention, the compound contains four or less hydrogen atoms.According to one embodiment of the present invention, the compound contains at least eight fluorine atoms, preferably at least ten fluorine atoms.

According to one embodiment of the present invention, at least one of Ar¹, Ar² and Ar³ comprise at least one moiety selected out of CF₃, CN and F, more preferred CF₃ and CN.

According to one embodiment of the present invention, at least one of Ar¹, Ar² and Ar³, preferably Ar¹ or at least Ar¹ is selected from one of the following moieties:

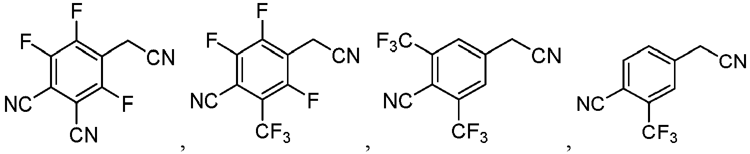

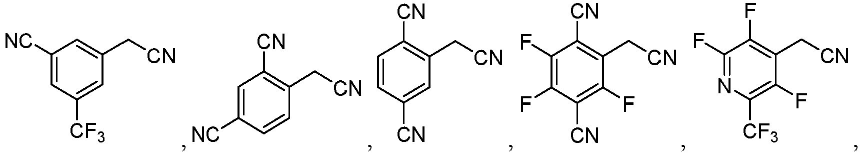

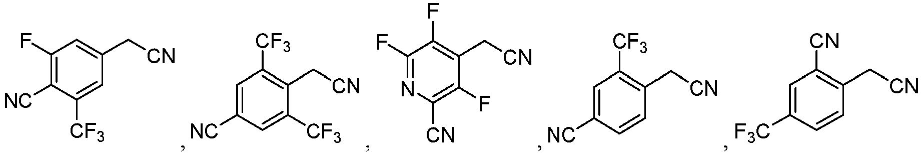

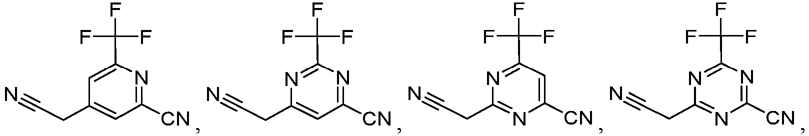

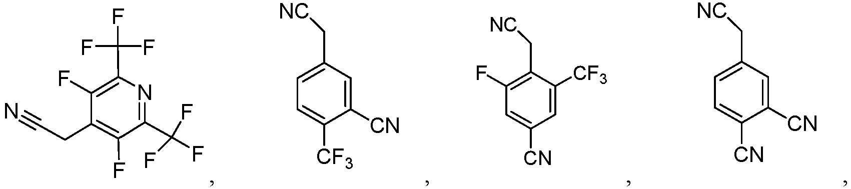

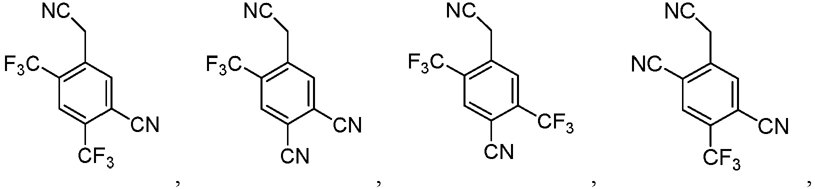

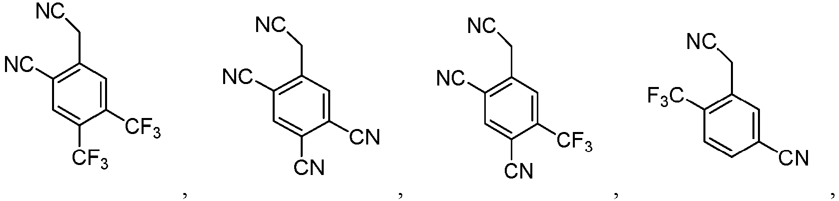

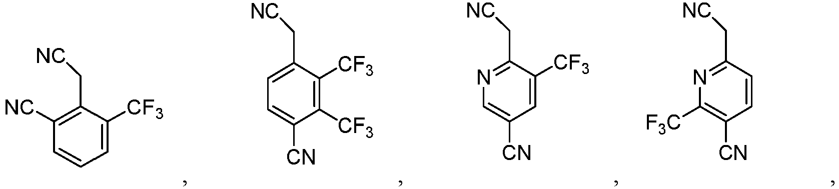

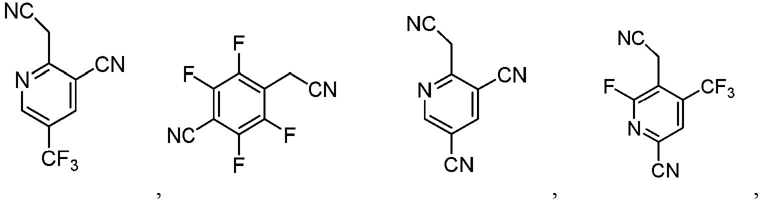

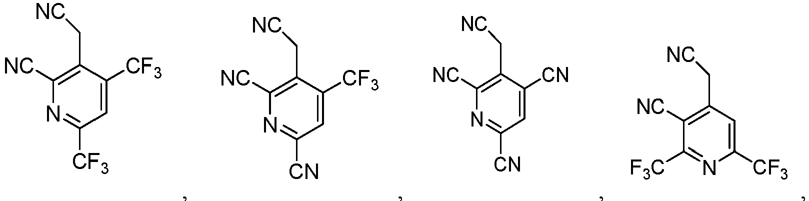

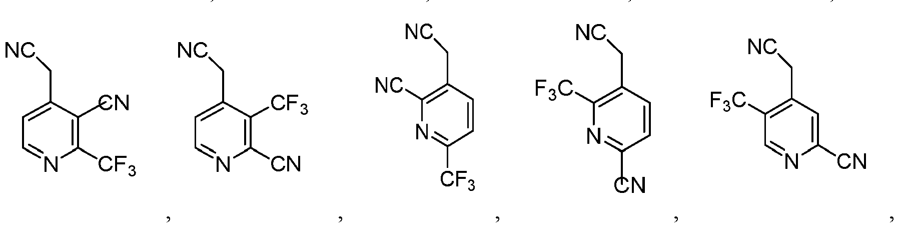

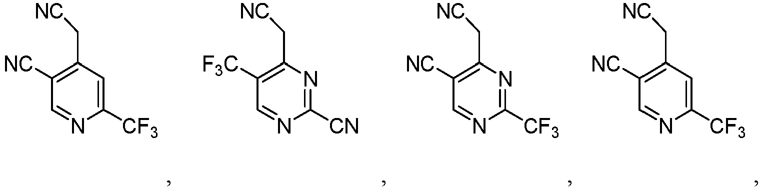

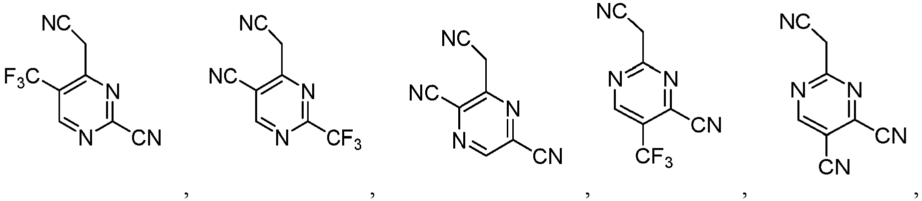

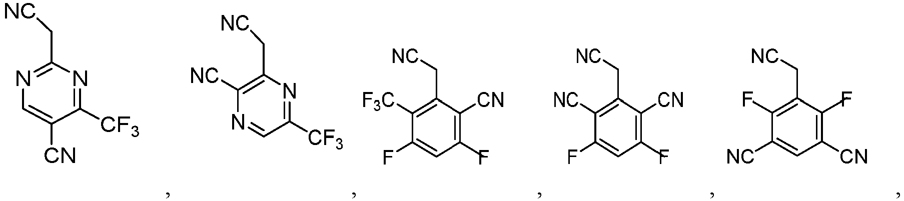

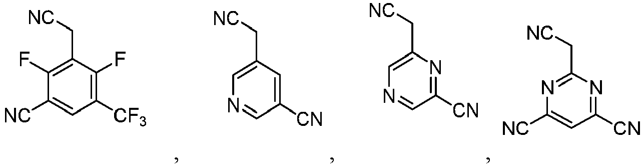

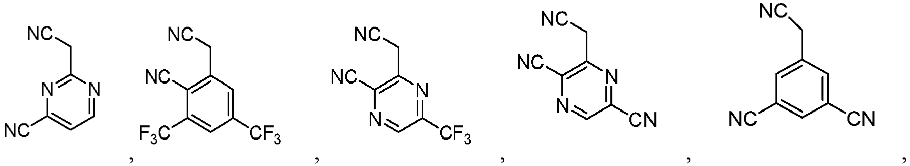

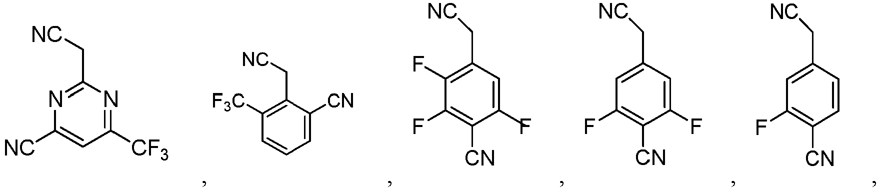

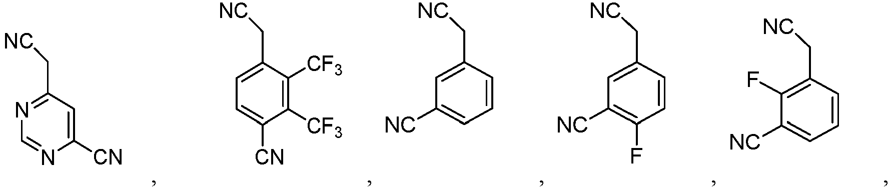

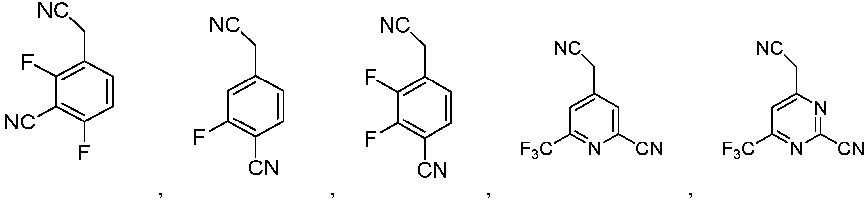

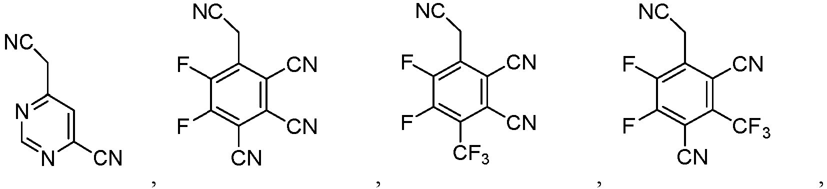

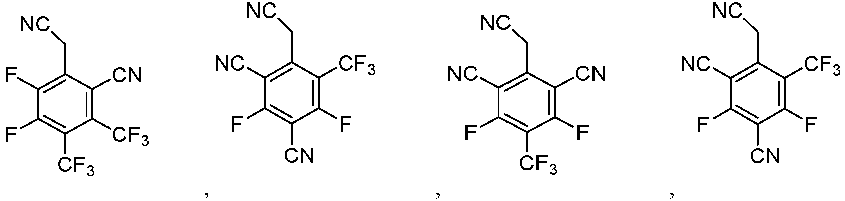

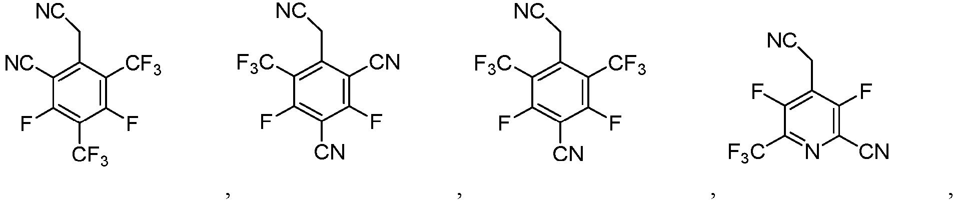

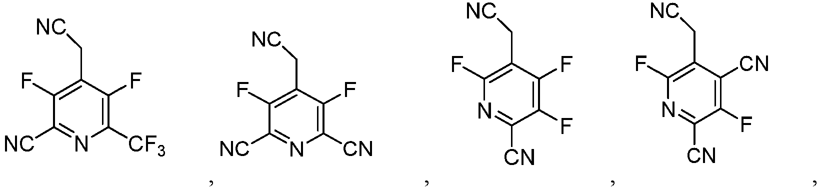

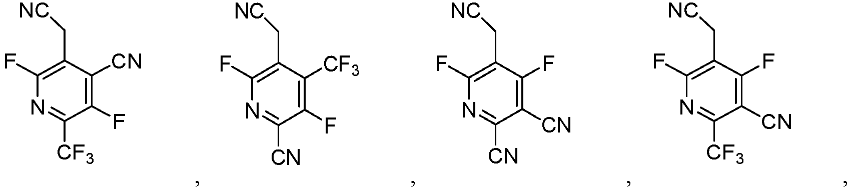

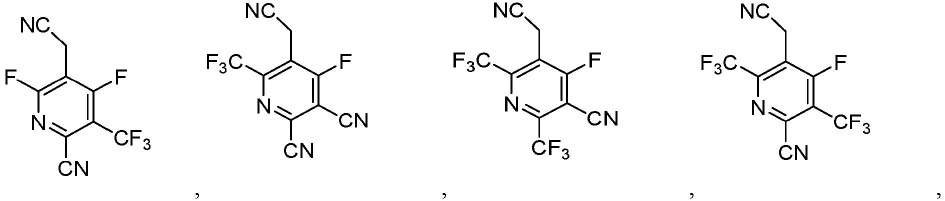

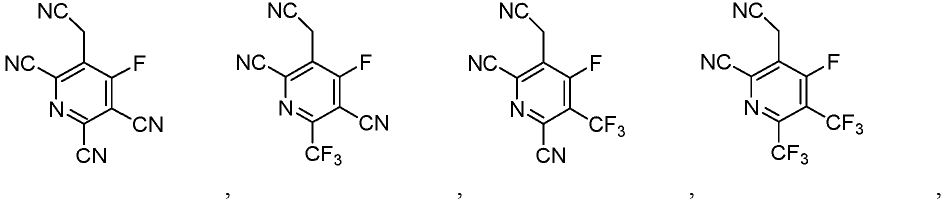

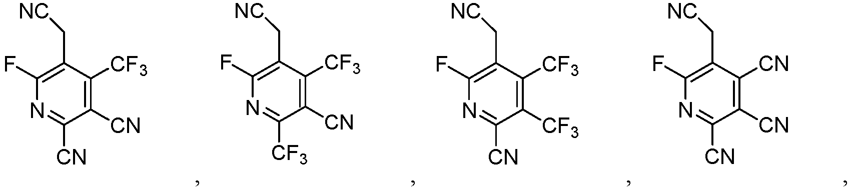

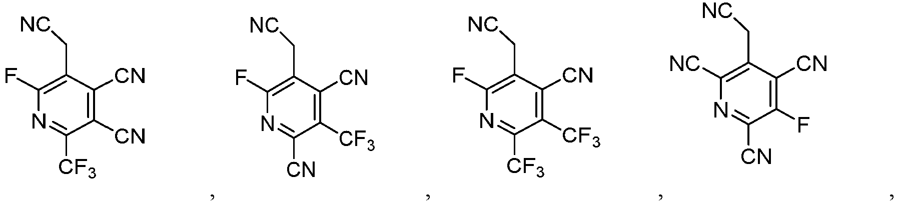

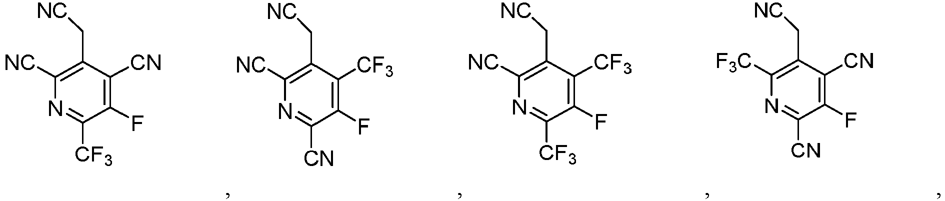

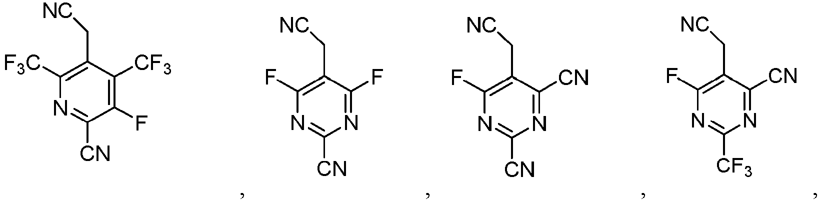

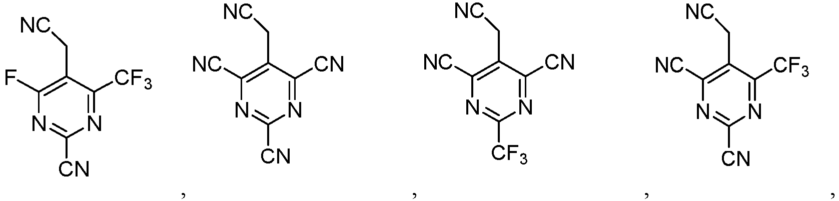

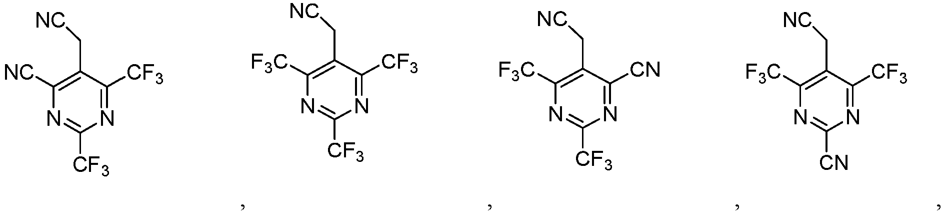

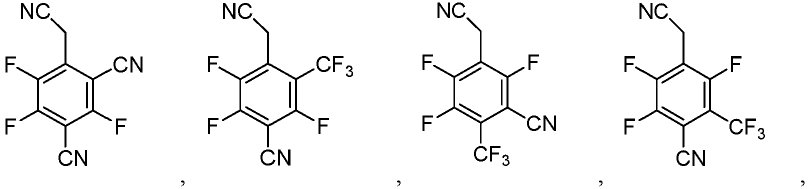

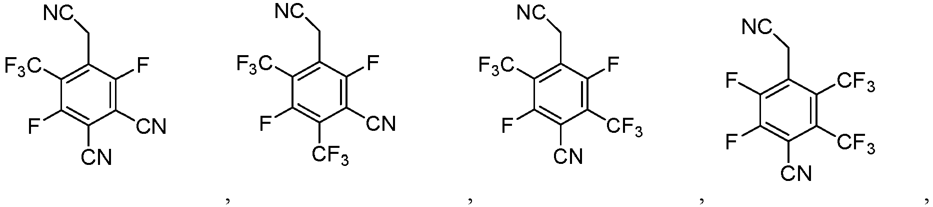

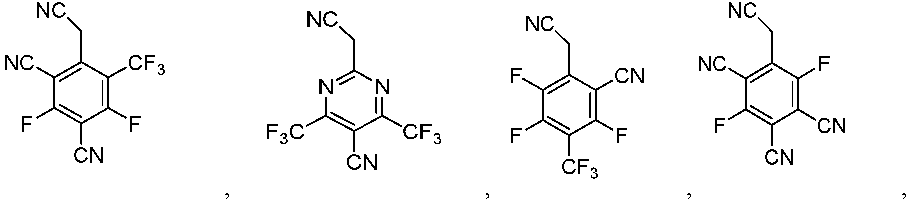

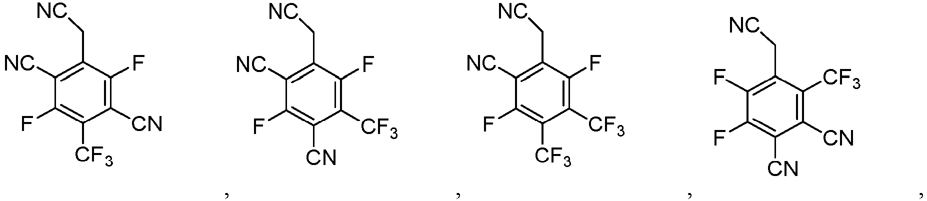

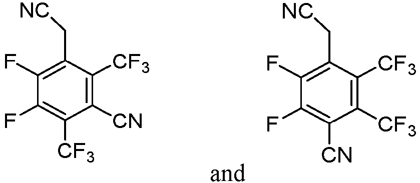

According to one embodiment of the present invention, at least two of A¹, A², and A³ are identical.

According to one embodiment of the present invention, A² and A³ are identical.

According to one embodiment of the present invention, A¹, A², and A³ are identical.

According to one embodiment of the present invention, A² is selected from

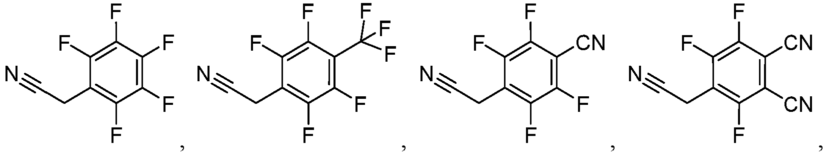

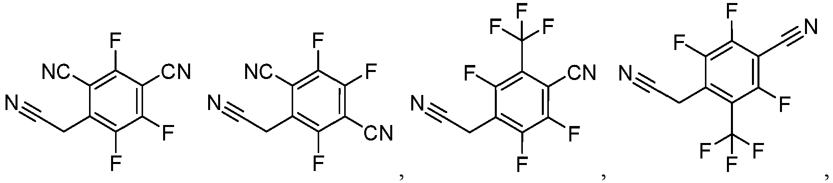

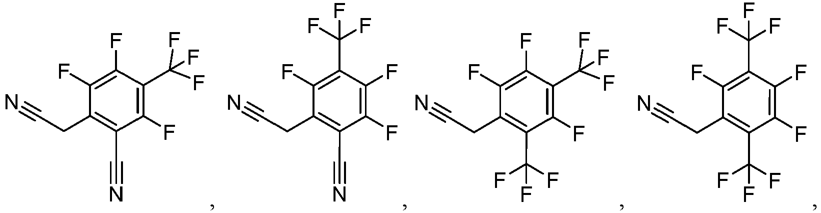

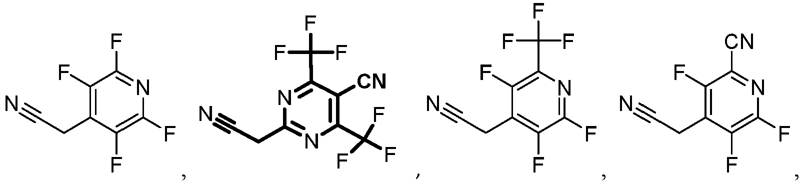

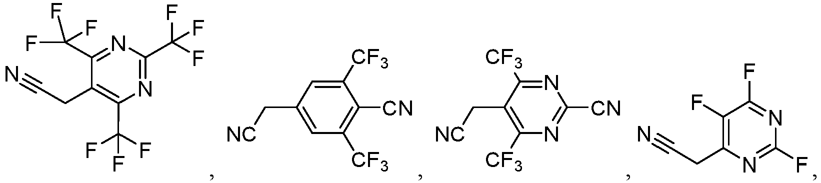

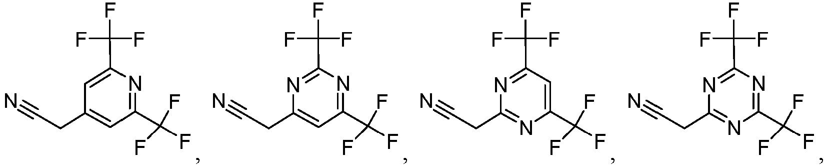

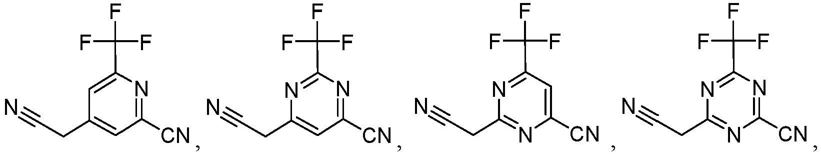

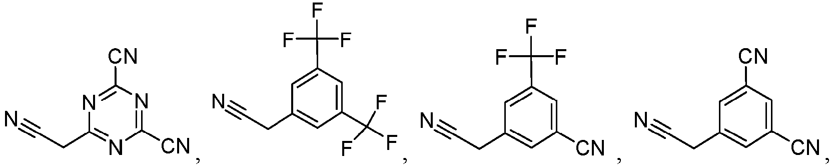

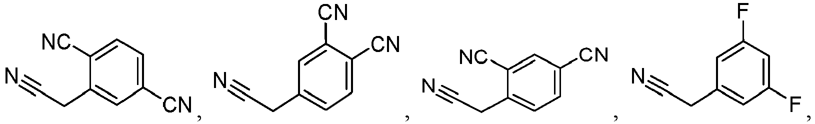

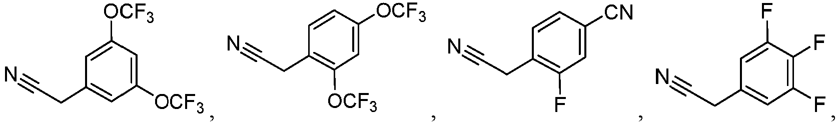

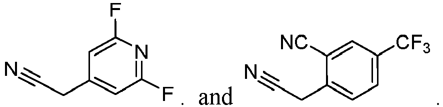

According to one embodiment of the present invention, A² and/or A³ are independently selected from

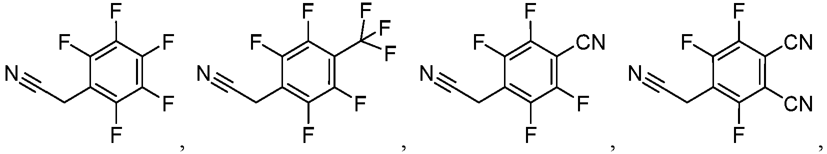

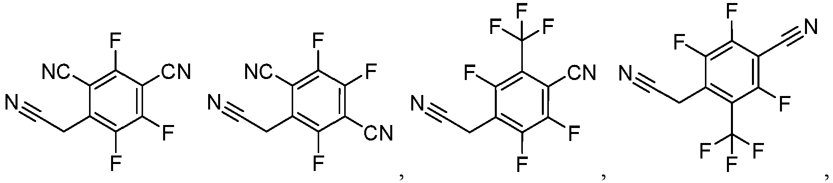

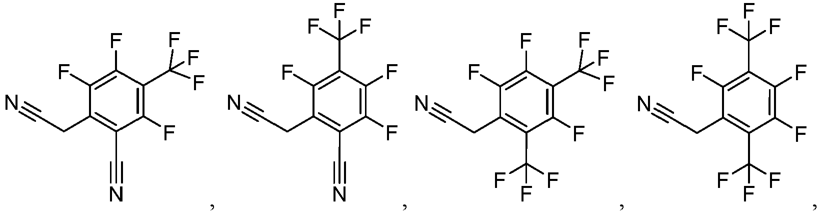

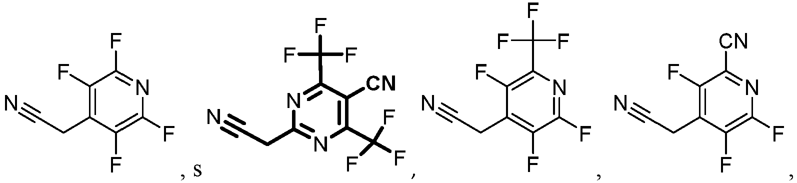

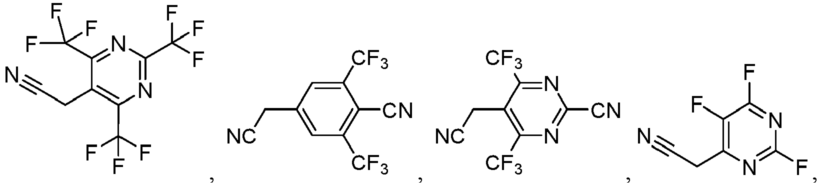

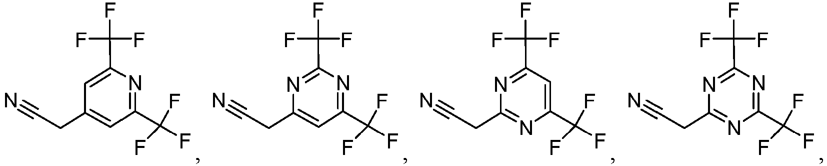

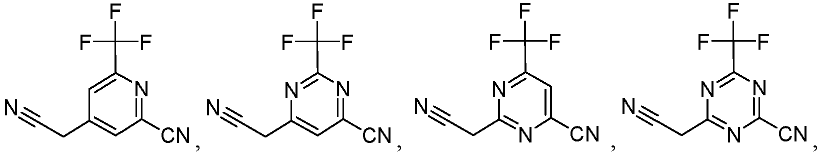

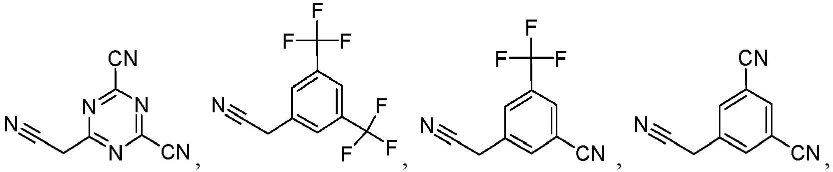

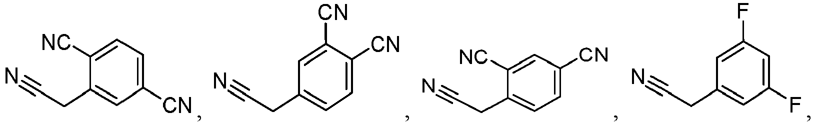

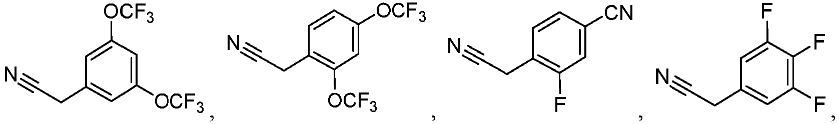

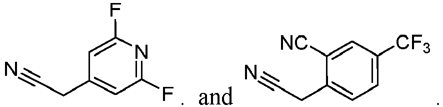

According to one embodiment of the present invention, the compound is selected of the formula (VI)

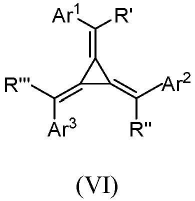

The present invention furthermore relates to a composition comprising a compound of formula (VI) and at least one compound of formula (VIa) to (VId)

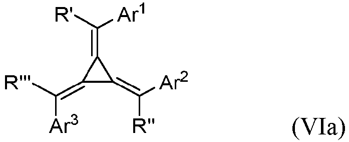

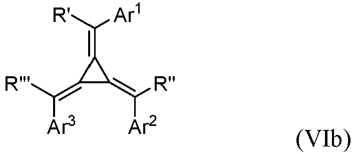

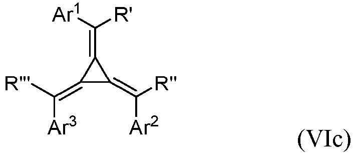

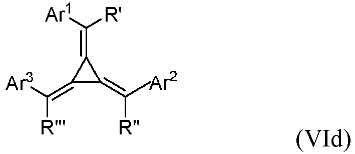

Another aspect of the present invention is related to an organic semiconductor layer comprising at least one compound according to the invention.

The present invention is also related to an organic electronic device comprising at least one compound according to the invention.

Another aspect of the present invention is related to an organic semiconductor layer comprising an organic semiconductor layer.

The present invention furthermore relates to an organic electronic device comprising an anode layer, a cathode layer and at least one organic semiconductor layer, wherein the at least one organic semiconductor layer comprises a compound according to the invention.

According to one embodiment, the organic electronic device comprising an anode layer, a cathode layer.

According to one embodiment, the organic electronic device further comprises a photoactive layer.

According to one embodiment, the organic electronic device comprising an anode layer, a cathode layer and at least one organic semiconductor layer, wherein the at least one organic semiconductor layer is arranged between the anode layer and the cathode layer; and wherein the at least one organic semiconductor layer comprises a compound according to the invention.

According to one embodiment, the organic semiconductor layer comprises a composition comprising a compound of formula (VI) and at least one compound of formula (VIa) to (VId) as described above.

**In** case the organic semiconductor layer comprises such a composition, throughout this application text the term "compound according to the invention" shall also intend to include the composition as described above.

Another aspect of the present invention provides an organic electronic device comprising an anode layer, a cathode layer, at least one organic semiconductor layer, and at least one photoactive layer, wherein the semiconductor layer is arranged between the cathode and the at least one photoactive layer, wherein the at least one organic semiconductor layer comprises a compound according to the invention.

Another aspect of the present invention provides an organic electronic device comprising an anode layer, a cathode layer and a charge generation layer, wherein the charge generation layer comprises a p-type charge generation layer and a n-type charge generation layer, wherein the p-type charge generation layer comprises a compound according to the invention.

According to one embodiment of the present invention the organic semiconductor layer and/or the compound according to the invention are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, the at least one organic semiconductor layer further comprises a substantially covalent matrix compound. Preferably, at least one semiconductor layer further comprising a substantially covalent matrix compound is arranged and/or provided adjacent to the anode layer.

According to a different aspect of the invention, a method of producing a compound of the invention is provided, comprising the steps of
a) Providing a compound of formula (I)
b) Heating the compound of formula (I) in a temperature range of ≥165 K for ≥ 10 min.

Preferably, step b) occurs in a temperature range of ≥Tₘ-50 K to ≤ Tₘ-10 K for ≥ 10 min, whereby Tₘ is the melting temperature of the compound of formula (I), more preferably at a temperature range of ≥Tₘ-40 K to ≤ Tₘ-20 K.

Preferably, step b) occurs in a temperature range of ≥T_{subl}-50 K to ≤ T_{subl}-10 K for ≥ 10 min, whereby T_{subl} is the sublimation temperature of the compound of formula (I), more preferably at a temperature range of ≥T_{subl}-40 K to ≤ T_{subl}-20 K.

Preferably, step b) occurs for ≥ 30 min to ≤ 2hrs.

Preferably, step b) occurs at reduced pressure, preferably at ≤10⁻¹ Pa, more preferably ≤ 10⁻² Pa, even more preferably ≤ 10⁻³ Pa and most preferred ≤ 10⁻⁴ Pa.

### Substantially covalent matrix compound

The organic semiconductor layer may further comprises a substantially covalent matrix compound. According to one embodiment the substantially covalent matrix compound may be selected from at least one organic compound. The substantially covalent matrix may consists substantially from covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

### Compound of formula (IX) or a compound of formula (X)

According to another aspect of the present invention, the at least one matrix compound, also referred to as "substantially covalent matrix compound", may comprises at least one arylamine compound, diarylamine compound, triarylamine compound, a compound of formula (IX) or a compound of formula (X): 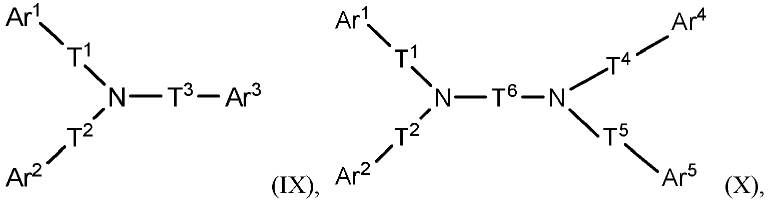 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(=O)R², CN, Si(R²)₃, P(=O)(R²)₂, OR², S(=O)R², S(=O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from D1 to D16: 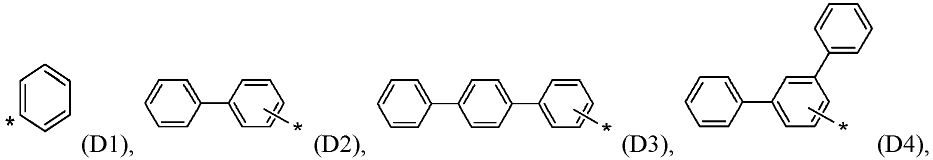 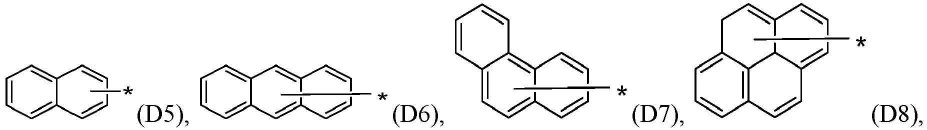 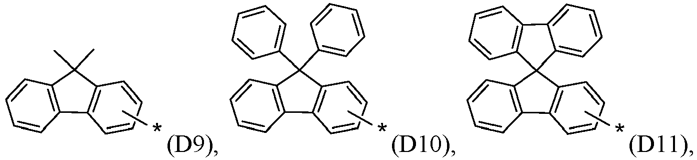 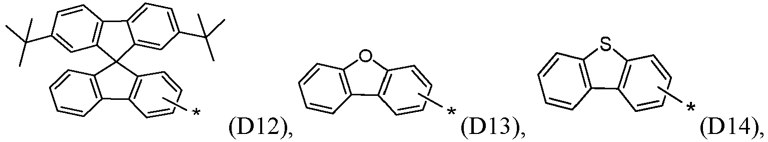 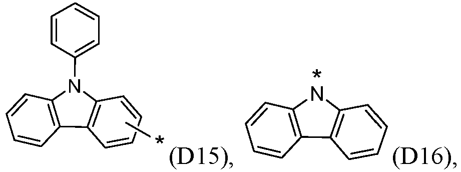 wherein the asterix "*" denotes the binding position.

According to an embodiment, wherein Ar¹, Ar², Ar³, Ar⁴ and Ar⁵ may be independently selected from D1 to D15; alternatively selected from D1 to D10 and D13 to D15.

According to an embodiment, wherein Ar¹, Ar², Ar³, Ar⁴ and Ar⁵ may be independently selected from the group consisting of D1, D2, D5, D7, D9, D10, D13 to D16.

The rate onset temperature may be in a range particularly suited to mass production, when Ar¹, Ar², Ar³, Ar⁴ and Ar⁵ are selected in this range.

The "matrix compound of formula (IX) or formula (X)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents are independently selected from methyl, phenyl or fluorenyl.

According to an embodiment of the electronic device, wherein the matrix compound of formula (IX) or formula (X) are selected from F1 to F 18:

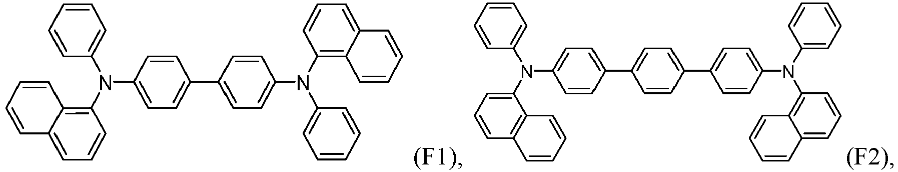

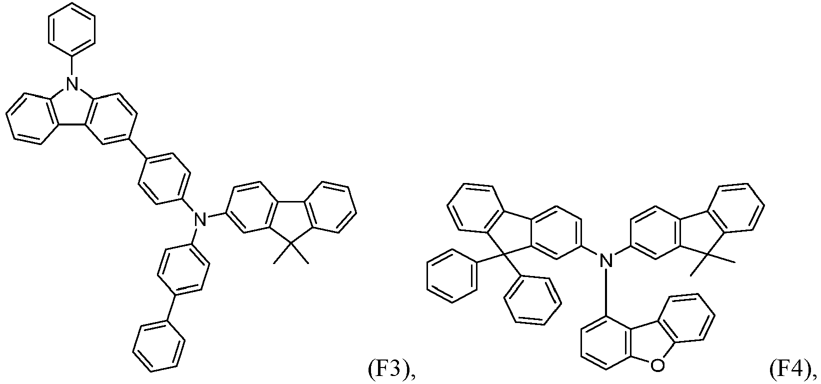

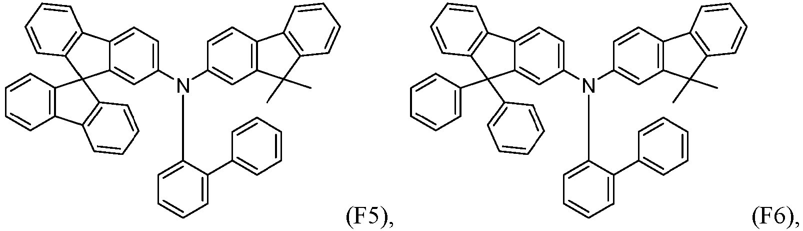

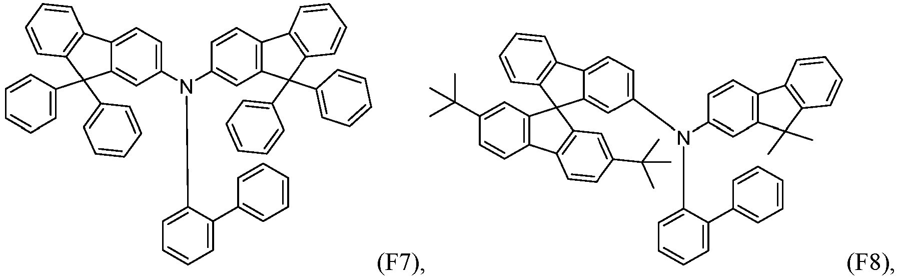

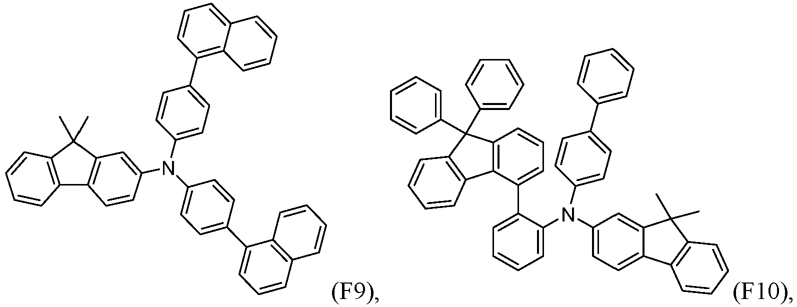

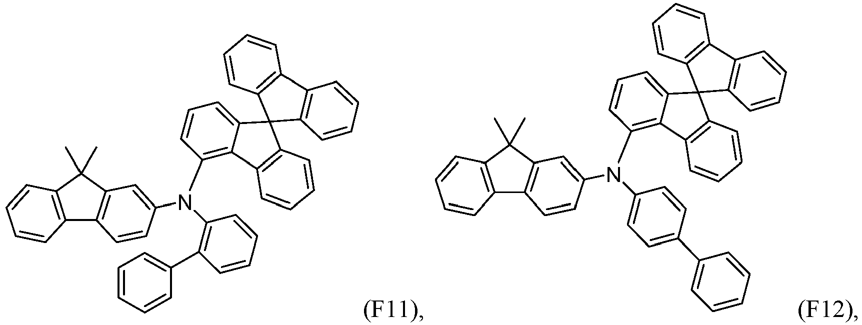

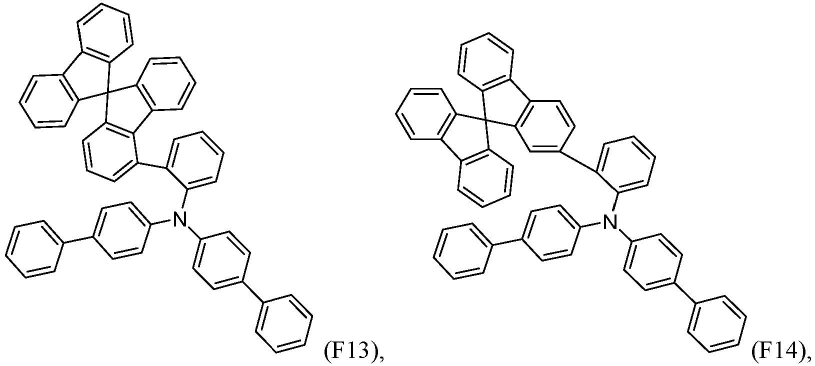

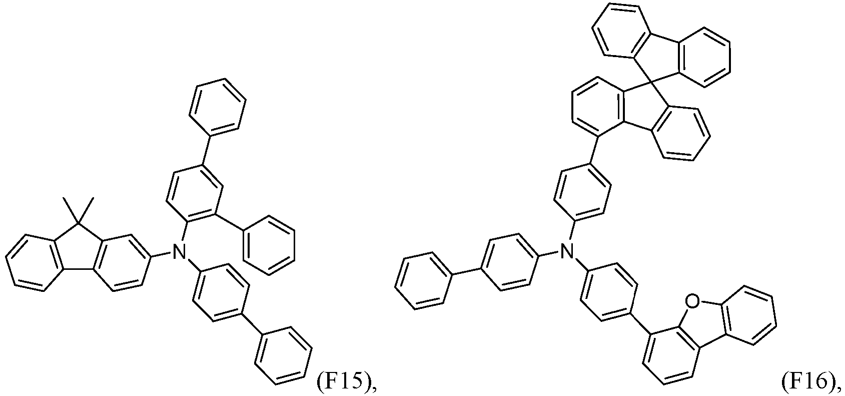

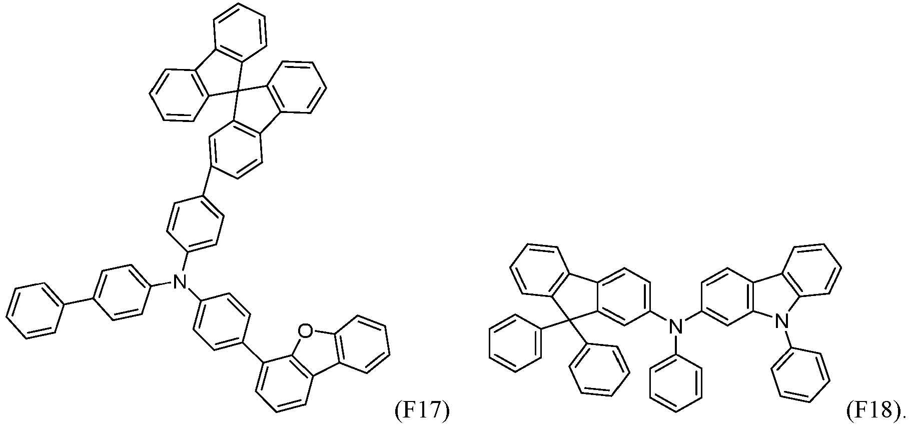

### Organic semiconductor layer

The organic semiconductor layer may be formed on the anode layer or cathode layer by vacuum deposition, spin coating, printing, casting, slot-die coating, Langmuir-Blodgett (LB) deposition, or the like. When the Organic semiconductor layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the organic semiconductor layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

The thickness of the organic semiconductor layer may be in the range from about 1 nm to about 20 nm, and for example, from about 2 nm to about 15 nm, alternatively about 2 nm to about 12 nm.

When the thickness of the organic semiconductor layer is within this range, the organic semiconductor layer may have excellent hole injecting and/or hole generation characteristics, without a substantial penalty in driving voltage.

According to one embodiment of the present invention, the organic semiconductor layer may comprise:
- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a compound according to the invention, and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the compound according to the invention is lower than the wt.-% of the substantially covalent matrix compound; wherein the weight-% of the components are based on the total weight of the organic semiconductor layer.

According to one embodiment of the invention, the organic electronic devices comprises at least one photoactive layer, wherein the photoactive layer is arranged between the anode layer and the cathode layer.

According to one embodiment of the invention, the organic electronic devices comprises at least one photoactive layer and at least one organic semiconductor layer is arranged between the anode and at least one photoactive layer.

According to one embodiment of the invention, the organic electronic devices comprises at least one photoactive layer and the at least one organic semiconductor layer is arranged between the anode and at least one photoactive layer.

According to one embodiment of the invention, the organic electronic devices comprises at least one photoactive layer and the at least one organic semiconductor layer is arranged between the anode and the at least one photoactive layer.

According to one embodiment of the invention, the organic electronic devices comprises at least one photoactive layer and at least one of the at least one organic semiconductor layer is arranged between the anode and at least one of the at least one photoactive layer.

According to one embodiment of the invention, the organic electronic device comprises at least one photoactive layer and the at least one of the at least one organic semiconductor layer is arranged between the anode and the at least one photoactive layer.

According to one embodiment of the invention, the organic electronic devices comprises at least one photoactive layer and the at least one organic semiconductor layers is arranged between the anode and the at least one photoactive layer.

According to one embodiment of the invention, the organic electronic device comprises at least two photoactive layers, wherein at least one organic semiconductor layers is arranged between the first and the second photoactive layer.

According to one embodiment of the invention, the organic electronic device comprises at least two photoactive layers, wherein at least one of the at least one organic semiconductor layers is arranged between the first and the second photoactive layer.

According to one embodiment of the invention, the organic electronic device comprises at least two photoactive layers, wherein one of the at least one organic semiconductor layers is arranged between the first and the second photoactive layer and one of the at least one organic semiconductor layers is arranged between the anode layer and the first photoactive layer.

According to one embodiment of the invention the electronic organic device is an electroluminescent device, preferably an organic light emitting diode.

The present invention furthermore relates to a display device comprising an organic electronic device according to the present invention.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode layer

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

According to a preferred embodiment the organic electrode device comprises an anode layer, whereby the anode layer comprises a first anode sub-layer and a second anode sub-layer, wherein
- the first anode sub-layer comprises a first metal having a work function in the range of ≥ 4 and ≤ 6 eV, and
- the second anode sub-layer comprises a transparent conductive oxide; and
- the second anode sub-layer is arranged closer to the hole injection layer.

According to one embodiment of the present invention, the first metal of the first anode sub-layer may be selected from the group comprising Ag, Mg, Al, Cr, Pt, Au, Pd, Ni, Nd, Ir, preferably Ag, Au or Al, and more preferred Ag.

According to one embodiment of the present invention, the first anode sub-layer has have a thickness in the range of 5 to 200 nm, alternatively 8 to 180 nm, alternatively 8 to 150 nm, alternatively 100 to 150 nm.

According to one embodiment of the present invention, the first anode sub-layer is formed by depositing the first metal via vacuum thermal evaporation.

It is to be understood that the first anode layer is not part of the substrate.

According to one embodiment of the present invention, the transparent conductive oxide of the second anode sub layer is selected from the group selected from the group comprising indium tin oxide or indium zinc oxide, more preferred indium tin oxide.

According to one embodiment of the present invention, the second anode sub-layer may has a thickness in the range of 3 to 200 nm, alternatively 3 to 180 nm, alternatively 3 to 150 nm, alternatively 3 to 20 nm.

According to one embodiment of the present invention, the second anode sub-layer may be formed by sputtering of the transparent conductive oxide.

According to one embodiment of the present invention, anode layer of the organic electronic device comprises in addition a third anode sub-layer comprising a transparent conductive oxide, wherein the third anode sub-layer is arranged between the substrate and the first anode sub-layer.

According to one embodiment of the present invention, the third anode sub-layer comprises a transparent oxide, preferably from the group selected from the group comprising indium tin oxide or indium zinc oxide, more preferred indium tin oxide.

According to one embodiment of the present invention, the third anode sub-layer may has a thickness in the range of 3 to 200 nm, alternatively 3 to 180 nm, alternatively 3 to 150 nm, alternatively 3 to 20 nm.

According to one embodiment of the present invention, the third anode sub-layer may be formed by sputtering of the transparent conductive oxide.

It is to be understood that the third anode layer is not part of the substrate.

According to one embodiment of the present invention, the hole injection layer is in direct contact with the anode layer.

### Hole injection layer

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise the same substantially covalent matrix compound as the organic semiconductor layer of the present invention.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

According to one embodiment of the present invention, the photoactive layer does not comprise the compound according to the invention.

The photoactive layer may be a light-emitting layer or a light-absorbing layer preferably a light-emitting layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment of the present invention, the emission layer does not comprise the compound according to the invention.

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)₃, and Btp₂lr(acac), but are not limited thereto. These compounds are phosphorescent emitters, however, fluorescent red emitter dopants could also be used.

Examples of phosphorescent green emitter dopants are Ir(ppy)₃ (ppy = phenylpyridine), Ir(ppy)₂(acac), Ir(mpyp)₃.

Examples of phosphorescent blue emitter dopants are F2Irpic, (F2ppy)₂Ir(tmd) and Ir(dfppz)₃ and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode layer

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

### Semiconductor layer

According to one embodiment the semiconductor layer comprises at least one compound according to the invention and at least one substantially covalent matrix.

According to one embodiment the semiconductor layer comprises at least one compound according to the invention is a hole injection layer.

### Charge generation layer

The term "n-type charge generation layer" is sometimes in the art also named n-CGL or electron generation layer and is intended to include the both.

The term "p-type charge generation layer" is sometimes in the art also named p-CGL or hole generation layer and is intended to include the both.

According to one embodiment of the present invention the p-type and/or n-type charge generation layer and/or the compound according to the invention are non-emissive.

According to one embodiment of the invention, the p-type charge generation layer is arranged closer to the cathode layer than the n-type charge generation layer.

According to one embodiment of the invention, the p-type charge generation layer further comprises a substantially covalent matrix compound.

### Organic light-emitting diode (OLED)

The organic electronic device according to the invention may be an organic light-emitting device.

According to one aspect of the present invention, there is provided an organic light-emitting diode (OLED) comprising: a substrate; an anode electrode formed on the substrate; an organic semiconductor layer comprising a compound according to the invention , a hole transport layer, an emission layer, an electron transport layer and a cathode electrode.

According to another aspect of the present invention, there is provided an OLED comprising: a substrate; an anode electrode formed on the substrate; an organic semiconductor layer comprising a compound according to the invention, a hole transport layer, an electron blocking layer, an emission layer, a hole blocking layer, an electron transport layer and a cathode electrode.

According to another aspect of the present invention, there is provided an OLED comprising: a substrate; an anode electrode formed on the substrate; an organic semiconductor layer comprising a compound according to the invention, a hole transport layer, an electron blocking layer, an emission layer, a hole blocking layer, an electron transport layer, an electron injection layer, and a cathode electrode.

According to another aspect of the present invention, there is provided an organic light-emitting diode (OLED) comprising: a substrate; an anode layer formed on the substrate; a charge generation layer according to invention, at least one emission layer and a cathode layer.

According to one aspect of the present invention, there is provided an organic light-emitting diode (OLED) comprising: a substrate; an anode layer formed on the substrate; a charge generation layer according to invention, at least a first and a second emission layers and a cathode layer, wherein the charge generation layer is arranged between the first and the second emission layer.

According to one aspect of the present invention, there is provided an organic light-emitting diode (OLED) comprising: a substrate; an anode layer formed on the substrate; a n-type charge generation layer, a p-type charge generation layer comprising a compound according to the invention,, a hole transport layer, an emission layer, an electron transport layer and a cathode layer.

According to another aspect of the present invention, there is provided an OLED comprising: a substrate; an anode layer formed on the substrate; a n-type charge generation layer, a p-type charge generation layer comprising a compound according to the invention, a hole transport layer, an electron blocking layer, an emission layer, a hole blocking layer, an electron transport layer and a cathode layer.

According to another aspect of the present invention, there is provided an OLED comprising: a substrate; an anode electrode formed on the substrate; a n-type charge generation layer, a p-type charge generation layer comprising a compound according to the invention, a hole transport layer, an electron blocking layer, an emission layer, a hole blocking layer, an electron transport layer, an electron injection layer, and a cathode electrode.

According to various embodiments of the present invention, there may be provided OLEDs layers arranged between the above mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

According to one embodiment of the invention, at least one semiconductor layer is arranged and/or provided adjacent to the anode layer.

According to one embodiment of the invention, at least one semiconductor layer of the present invention is a hole-injection layer.

According to another aspect, the at least one semiconductor layer may have a layer thickness of at least about ≥ 0.5 nm to about ≤ 10 nm, preferably of about ≥ 2 nm to about ≤ 8 nm, also preferred of about ≥ 3 nm to about ≤ 5 nm.

The organic semiconductor layer according to the invention may be the first hole injection layer and/or the p-type charge generation layer.

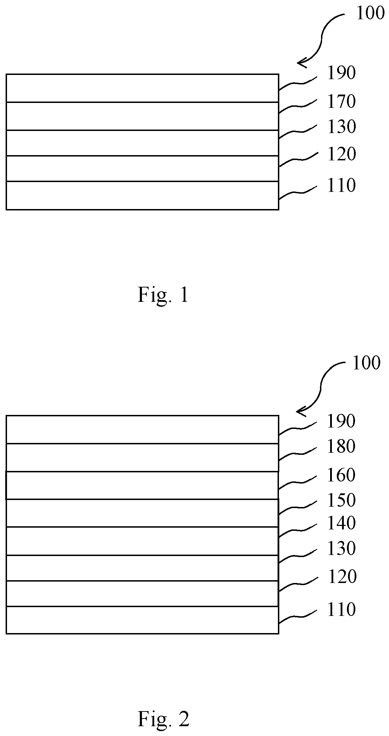

For example, the OLED according to Fig. 2 may be formed by a process, wherein on a substrate (110), an anode layer (120), a hole injection layer (130), a hole transport layer (140), an electron blocking layer (145), an emission layer (150), a hole blocking layer (155), an electron transport layer (160), an electron injection layer (180) and the cathode layer (190) are subsequently formed in that order.

### Organic electronic device

The organic electronic device according to the invention may be a light emitting device, thin film transistor, a battery, a display device or a photovoltaic cell, preferably a light emitting device or a photovoltaic cell, and most preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound according to the invention according to the invention, and
- a second deposition source to release the substantially covalent matrix compound;
   the method comprising the steps of forming the organic semiconductor layer; whereby for an organic light-emitting diode (OLED):
- the organic semiconductor layer is formed by releasing the compound according to the invention according to the invention from the first deposition source and the substantially covalent matrix compound from the second deposition source.

According to various embodiments of the present invention, the method may further include forming on the anode electrode, at least one layer selected from the group consisting of forming a hole transport layer or forming a hole blocking layer, and an emission layer between the anode electrode and the first electron transport layer.

According to various embodiments of the present invention, the method may further include the steps for forming an organic light-emitting diode (OLED), wherein
- on a substrate an anode electrode is formed,
- on the anode electrode an organic semiconductor layer comprising a compound according to the invention is formed,
- on the organic semiconductor layer comprising a compound according to the invention a hole transport layer is formed,
- on the hole transport layer an emission layer is formed,
- on the emission layer an electron transport layer is formed, optionally a hole blocking layer is formed on the emission layer,
- and finally a cathode electrode is formed,
- optional a hole blocking layer is formed in that order between the first anode electrode and the emission layer,
- optional an electron injection layer is formed between the electron transport layer and the cathode electrode.

According to various embodiments of the present invention, there is provided a method using:
- a first deposition source to release the compound according to the invention according to the invention, and
- a second deposition source to release the substantially covalent matrix compound;
- a third deposition source to release the n-CGL matrix compound;
- a fourth deposition source to release the n-CGL dopant;

the method comprising the steps of forming the p-type charge generation layer; whereby for an organic light-emitting diode (OLED):
   - the p-type charge generation layer is formed by releasing the compound according to the invention according to the invention from the first deposition source and the substantially covalent matrix compound from the second deposition source;
the method comprising the steps of forming the n-type charge generation layer; whereby for an organic light-emitting diode (OLED):
   - the n-type charge generation layer is formed by releasing the n-CGL matrix compound according to the invention from the third deposition source and n-CGL dopant from the fourth deposition source.

According to one embodiment of the present invention, there is provided a method of manufacturing an organic electronic device, by the following steps:
(i) providing a surface;
(ii) providing a compound according to the invention;
(iii) providing one deposition source for release of the compound according to the invention;
(iv) evaporating the compound according to the invention at an elevated temperature and at a reduced pressure forming the organic compound in a gaseous phase; and
(v) depositing the organic compound in the gaseous phase on the surface.

According to one embodiment of the present invention, there is provided a method of manufacturing an organic electronic device, by the following steps:
(i) providing a surface;
(ii) providing a compound according to the invention;
(iii) providing one deposition source for release of the compound according to the invention;
(iv) evaporating the compound according to the invention at an elevated temperature and at a reduced pressure forming the organic compound in a gaseous phase;
(v) depositing the organic compound in the gaseous phase on the surface; and
(vi) forming an organic semiconductor layer.

According to one embodiment of the present invention, there is provided a method of manufacturing an organic electronic device, by the following steps:
(i) providing a surface;
(ii) providing a compound according to the invention;
(iib) providing a substantially covalent matrix compound;
(iii) providing one deposition source for release of the compound according to the invention;
(iiib)providing a further deposition source to release the substantially covalent matrix compound;
(iv) evaporating the compound according to the invention and the substantially covalent matrix compound at an elevated temperature and at a reduced pressure forming the organic compound in a gaseous phase; and
(v) depositing the organic compound in the gaseous phase on the surface.

According to one embodiment of the present invention, there is provided a method of manufacturing an organic electronic device, by the following steps:
(i) providing a surface;
(ii) providing a compound according to the invention;
(iib) providing a substantially covalent matrix compound;
(iii) providing one deposition source for release of the compound according to the invention;
(iiib)providing a second deposition source to release the substantially covalent matrix compound;
(iv) evaporating the compound according to the invention and the substantially covalent matrix compound at an elevated temperature and at a reduced pressure forming the organic compound in a gaseous phase;
(v) depositing the organic compound in the gaseous phase on the surface; and
(vi) forming an organic semiconductor layer, wherein the organic semiconductor layer is formed by releasing the compound according to the invention according to the invention from the deposition source and the substantially covalent matrix compound from the further deposition source.

According to one embodiment of the present invention, the evaporation temperature in step (iv) can be ≥100 °C, ≥110°C, ≥120°C, ≥130°C, ≥140°C, preferably ≥150°C, more preferably 160°C and most preferably 165°C.

According to one embodiment of the present invention, the evaporation temperature in step (iv) can be in the range of 100°C to 300°C, in the range of 110°C to 300°C, in the range of 120°C to 300°C, in the range of 130°C to 300°C, in the range of 140°C to 300°C, preferably in the range of 150°C to 300°C, more preferably in the range of 160°C to 300°C, and even more preferably 165°C to 300°C.

According to one embodiment of the present invention, the pressure in step (iv) is preferably less than 10⁻¹ Pa, more preferably less than less than 10⁻² Pa, even more preferably less than 10⁻³ Pa, most preferably less than 10⁻⁴ Pa.

According to one embodiment of the present invention, duration of the evaporation step (iv) is preferably, longer than 100 hours, more preferably longer than 150 hours, even more preferably longer than 200 hours.According to various embodiments, the OLED may have the following layer structure, wherein the layers having the following order:
anode, organic semiconductor layer comprising a compound according to the invention according to the invention, first hole transport layer, second hole transport layer, emission layer, optional hole blocking layer, electron transport layer, optional electron injection layer, and cathode.

According to various embodiments of the present invention, the method may further include forming on the anode electrode, at least one layer selected from the group consisting of forming a hole transport layer or forming a hole blocking layer, an emission layer and a n-type charge generation layer between the anode electrode and the cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a schematic sectional view of an organic electronic device, according to an exemplary embodiment of the present invention;
FIG. 2 is a schematic sectional view of an organic light-emitting diode (OLED), according to an exemplary embodiment of the present invention;
FIG. 3 is a schematic sectional view of an OLED, according to an exemplary embodiment of the present invention.
FIG. 4 is a schematic sectional view of an OLED, according to an exemplary embodiment of the present invention.
FIG. 5 is a schematic sectional view of an OLED, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate 110, an anode layer 120 and a hole injection layer (HIL) 130 which may comprise a compound according to the invention. The HIL 130 is disposed on the anode layer 120. Onto the HIL 130, a photoactive layer (PAL) 170 and a cathode layer 190 are disposed.

FIG. 2 is a schematic sectional view of an organic light-emitting diode (OLED) 100, according to an exemplary embodiment of the present invention. The OLED 100 includes a substrate 110, an anode layer 120 and a hole injection layer (HIL) 130 which may comprise a compound according to the invention. The HIL 130 is disposed on the anode layer 120. Onto the HIL 130, a hole transport layer (HTL) 140, an emission layer (EML) 150, an electron transport layer (ETL) 160, an electron injection layer (EIL) 180 and a cathode layer 190 are disposed. Instead of a single electron transport layer 160, optionally an electron transport layer stack (ETL) can be used.

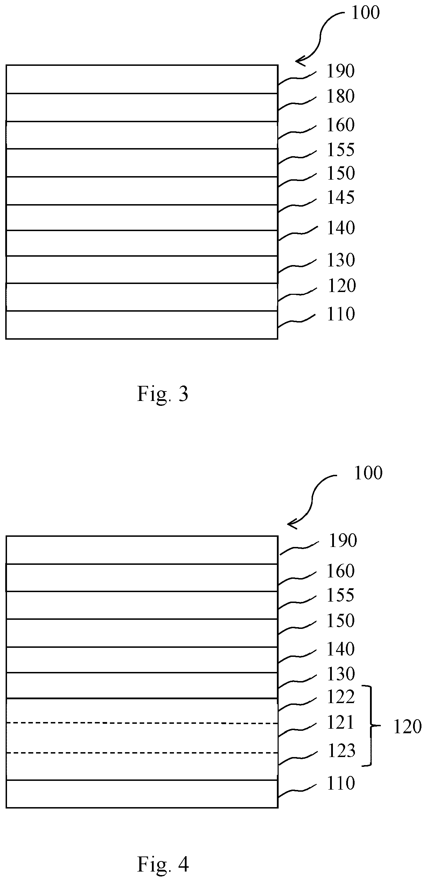

FIG. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode layer 120, a hole injection layer (HIL) 130 which may comprise a compound according to the invention, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode layer 190.

FIG. 4 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, an hole transport layer (HTL) 140, a first emission layer (EML) 150, a hole blocking layer (HBL) 155, an electron transport layer (ETL) 160, and a cathode layer 190 are disposed. The hole injection layer 130 may comprise a compound according to the invention.

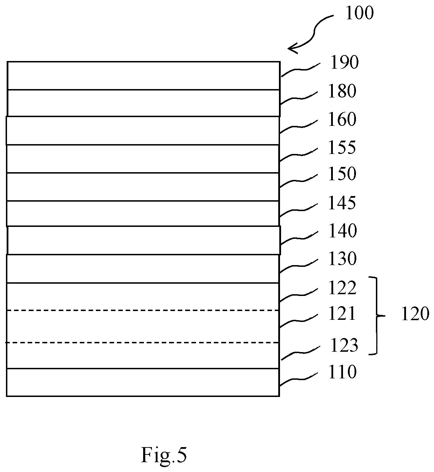

FIG. 5 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, a hole transport layer (HTL) 140, an electron blocking layer (EBL) 145, a first emission layer (EML) 150, a hole blocking layer (HBL) 155, an electron transport layer (ETL) 160, an electron injection layer (EIL) 180 and a cathode layer 190 are disposed. The hole injection layer 130 may comprise a compound according to the invention.

While not shown in Fig. 1 to Fig. 5, a capping and/or sealing layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Detailed description

The invention is furthermore illustrated by the following examples which are illustrative only and non-binding.

### Thermal annealing

Up to 15 g compound are loaded into the evaporation source of a sublimation apparatus. The sublimation apparatus consists of an inner glass tube consisting of bulbs with 46 mm diameter which are placed inside a glass tube with 50 mm diameter. The sublimation apparatus is placed inside a tube oven (DSU 20 from Creaphys). The sublimation apparatus is evacuated via a primary pump (Vacuubrand Type MD12C NT) and a turbomolecular pump (Pfeiffer Vacuum Type HiPace 80). The pressure is measured between the sublimation apparatus and the turbomolecular pump using a full-range pressure gauge (Pfeiffer Vacuum Type PKR 251). When the pressure has been reduced to 10⁻⁵ mbar, the temperature is increased with 2K/min to the desired annealing temperature. Upon reaching described conditions, material is left dwelling for minimum 1 hour followed by shut down of tube oven. Subsequent to cooling down to room temperature sublimation apparatus is vented with dry nitrogen up to ambient pressure. Material is harvested from evaporation source of sublimation apparatus.

### DSC

The absence of a glass transition temperature (Tg) is measured under nitrogen and using a heating rate of 10 K per min in a Mettler Toledo DSC 822e differential scanning calorimeter as described in DIN EN ISO 11357, published in March 2010.

### HV-TGA 5% mass loss temperature

10 mg compound are loaded into 2 ccm Al₂O₃ crucible, which is mounted in high vacuum -thermogravimetric analysis (HV-TGA) setup. The HV-TGA setup consists of an evaporation source (Creaphys DE-2-CF40), a thermocouple (Thermo Sensor GmbH NiCr-Ni, Typ K) placed inside the crucible and a quartz crystal microbalance (QCM, Inficon 750-1000-G10, 6 MHz). The HV-TGA setup is part of vacuum chamber system equipped with a scroll pump, a turbomolecular pump, a nitrogen inlet with mass flow controller and a progressive valve between scroll and turbomolecular pump. The combination of nitrogen inlet and pump valve allows pressures of 1e 2 mbar to 1e-6 mbar, whereas standard operational pressure is 1e-4 mbar with a stability of +/- 10%. Subsequent to reaching desired pressure the evaporation source temperature is ramped from room temperature to 600 °C at a rate of 10 °C/min. The compound is fully evaporated and detected by the QCM. The frequency shift of the QCM during the whole temperature ramp corresponds to a mass loss of 100%.

The reference temperature for dopant materials is taken at a mass loss of 5% as the obtained values have closest match to processing temperature in linear evaporation sources at mass production.

### Technical Effect of the invention

In the following several comparative compounds and inventive compounds were investigated. The structure of the comparative compounds is given in the follosing Table 1, They were prepared using standard procedurs, e.g. as described in EP2180029A1 and WO2016097017A1.

**Table 1: Structure of several comparative compounds**

| Comparative example No. | Structure |
|---|---|
| Comparative example No. 1 | 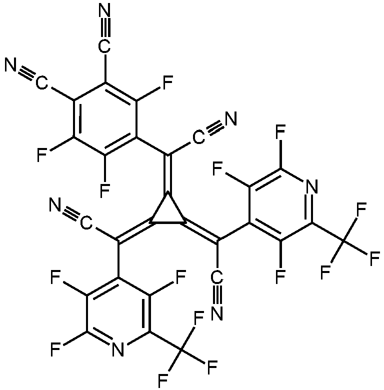 |
| Comparative example No. 2 | 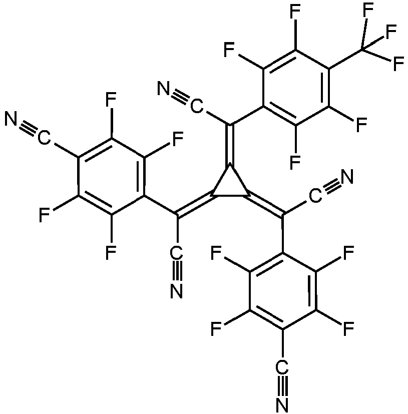 |
| Comparative Example No. 3 | 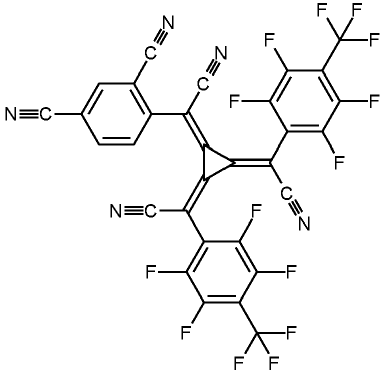 |
| Comparative Example No. 4 | 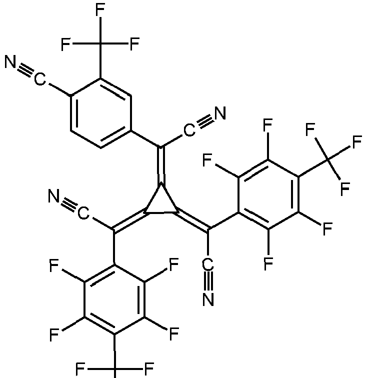 |
| Comparative Example No. 5 | 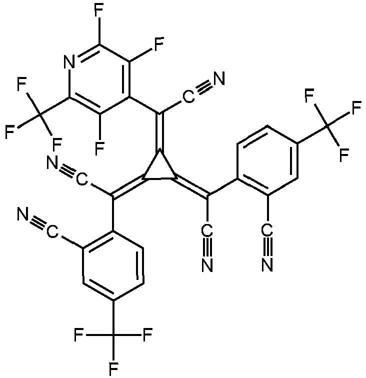 |
| Comparative Example No. 6 | 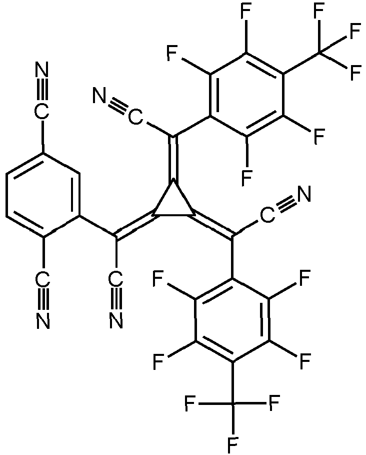 |
| Comparative Example No. 7 | 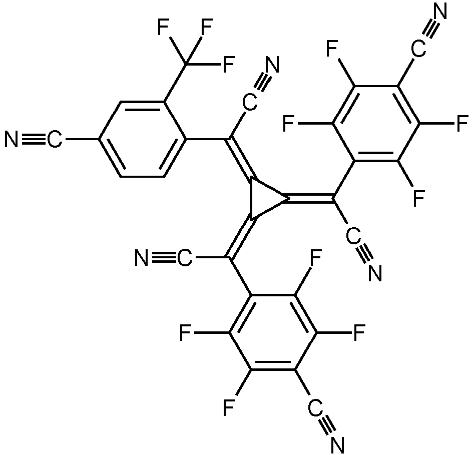 |

From these comparative examples, the inventive examples were prepared as follows, using the above described general method.

**Table 2: Thermal annealing details for inventive examples 1 to 7.**

| **Starting compound.** | **Thermal annealing procedure details** |
|---|---|
| Comparative example No. 1 | 1h at 180°C |
| Comparative example No. 2 | 1h at 180 °C |
| Comparative example No. 3 | 1h at 190 °C |
| Comparative example No. 4 | 1h at 179 °C |
| Comparative example No. 5 | 1h at 180 °C |
| Comparative example No. 6 | 1h at 192°C |
| Comparative example No. 7 | 1h at 200°C |

No substantial sublimation was observed for any of the compounds.

Table 3 lists several physical properties of the comparative and inventive examples,

**Table 3: Several physical properties of the comparative and inventive examples**

| | **Tg (°C)** | **Tm (°C)** | **LUMO [eV]** | **HV-TGA5%** |
|---|---|---|---|---|
| Inventive example 1 | n/obs | 224 | -5.33 | 190 |
| Comparative example 1 | 112 | n/obs | -5,33 | 168 |
| Inventive example 2 | n/obs | 236 | -5,10 | 197 |
| Comparative example 2 | 112 | 236 | -5,10 | 173 |
| Inventive example 3 | n/obs | 238 | -4,92 | 185 |
| Comparative example 3 | 109 | 238 | -4,92 | 174 |
| Inventive example 4 | n/obs | 267 | -4,95 | 210 |
| Comparative example 4 | 91 | 267 | -4,95 | 187 |
| Inventive example 5 | n/obs | 227 | -4,91 | 199 |
| Comparative example 5 | 100 | 227 | -4,91 | 174 |
| Inventive example 6 | n/obs | 225 | -4,93 | 210 |
| Comparative example 6 | 112 | n/obs | -4.93 | 179 |
| Inventive example 7 | n/obs | 220 | -5.07 | 214 |
| Comparative Example 7 | 120 | n/obs | -5.07 | 202 |

It is apparent that compared to the comparative compounds, the HV-TGA 5% is improved. Thus, the compounds according to the invention may be beneficial for reducing tool contamination during the manufacturing of an organic electronic device.

The particular combinations of elements and features in the above detailed embodiments are exemplary only. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims. Furthermore, reference signs used in the description and claims do not limit the scope of the invention as claimed.

## Claims

1. A compound of formula (I) 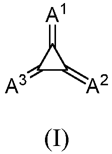
wherein A¹, A² and A³ are represented by formula (II), (III), (IV), respectively 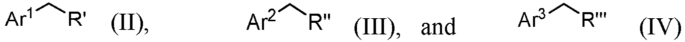
whereby Ar¹, Ar² and Ar³ are independently selected from substituted or unsubstituted aryl, substituted or unsubstituted C₆ to C₂₄ aryl, substituted or unsubstituted heteroaryl or C₂ to C₂₄ heteroaryl, wherein the substituents on Ar¹, Ar² and Ar³ are independently selected form an electron-withdrawing group, NO₂, CN, halogen, Cl, F, partially fluorinated or perfluorinated alkyl, partially fluorinated or perfluorinated C₁ to C₁₂ alkyl, CF3, partially fluorinated or perfluorinated alkoxy, partially fluorinated or perfluorinated C₁ to C₆ alkoxy or D;
R', R" and R‴ are independently selected from substituted or unsubstituted aryl, substituted or unsubstituted C₆ to C₁₈ aryl, substituted or unsubstituted heteroaryl, substituted or unsubstituted C₂ to C₁₈ heteroaryl, electron-withdrawing group, partially fluorinated or perfluorinated alkyl, partially fluorinated or perfluorinated C₁ to C₆ alkyl, halogen, F, CN, and NO₂;
wherein in formulae (II) to (IV) each Ar¹, Ar² and Ar³ with the corresponding R', R'' and R‴ can form a substituted or unsubstituted carbocyle or, preferably a substituted or unsubstituted heterocycle, wherein the substituents can be electron-withdrawing group, F, CN, perfluorinated C₁ to C₈ alkyl, substituted or unsubstituted C₆ to C₁₈ aryl, substituted or unsubstituted C₂ to C₁₈ heteroaryl, substituted or unsubstituted carbocyclene, substituted or unsubstituted carbocyclidene, substituted or unsubstituted alkylene, substituted or unsubstituted alkylidene;
wherein the compound of formula (I) does not exhibit a glass transition temperature when measured by DSC during heating at 10 K/min.

2. The compound of claim 1, whereby the compound has been heated to ≥ 150 °C.

3. The compound of claim 1 or 2, having a LUMO energy level, expressed in the absolute scale referring to vacuum energy level being zero of ≤ -4.5 eV calculated using the hybrid functional B3LYP with a Gaussian 6-31G* basis set as implemented in the program package TURBOMOLE V6.5.

4. The compound of any of the claims 1 to 3, wherein the compound has a melting temperature Tₘ of ≥200 °C.

5. The compound of any of the claims 1 to 4, wherein the compound has a melting enthalpy ΔHₘ, of ≥ 30 kJ/mol.

6. The compound of any of the claims 1 to 5, whereby the compound has four to eight CN- groups.

7. The compound of any of the claims 1 to 6, whereby the compound has a molecular mass of ≥380 g/mol.

8. The compound any of the claims 1 to 7, whereby at least one of the R', R'' and R‴ is CN.

9. The compound of any of the claims 1 to 8, wherein at least one of the Ar¹, Ar² and Ar³ is selected from Formula (V): 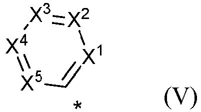
wherein X¹ is selected from CR¹ or N;
X² is selected from CR² or N;
X³ is selected from CR³ or N;
X⁴ is selected from CR⁴ or N;
X⁵ is selected from CR⁵ or N;
wherein R¹, R², R³, R⁴, and R⁵ are independently selected from electron-withdrawing group, NO₂, CN, halogen, Cl, F, partially fluorinated or perfluorinated alkyl, partially fluorinated or perfluorinated C₁ to C₁₀ alkyl, CF₃, partially fluorinated or perfluorinated alkoxy, partially fluorinated or perfluorinated C₁ to C₆ alkoxy, D or H;
wherein at least two of X¹ to X⁵ are independently selected from CR¹ to CR⁵; and
wherein the asterix "*" denotes the binding position.

10. An organic semiconductor layer comprising at least one compound of formula (I) according to any of the preceding claims 1 to 9.

11. An organic electronic device comprising at least one organic semiconductor layer of claim 10.

12. The organic electronic device of claim 11, whereby the organic electronic device comprises an anode layer, a cathode layer, at least one photoactive layer and at least one organic semiconductor layer, wherein the at least one organic semiconductor layer is arranged between the anode layer and at least one photoactive layer.

13. The organic electronic device of any of the claims 11 to 12, whereby the organic electronic device comprises at least two photoactive layers, wherein at least one organic semiconductor layer is arranged between the first and the second photoactive layer.

14. The organic electronic device of any of the claims 11 to 13, whereby the electronic organic device is a light emitting device, a thin film transistor, a battery, a display device or a photovoltaic cell, and preferably a light emitting device, preferably an organic light emitting diode.

15. A display device comprising an organic electronic device according to any of the claims 11 to 14.

## Patentansprüche

1. Eine Verbindung der Formel (I), 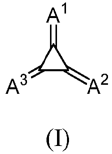
wobei A¹, A² and A³ durch die Formel (II), (III) bzw. (IV) dargestellt sind, 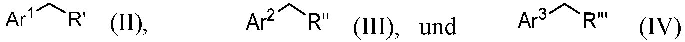
wobei Ar¹, Ar² und Ar³ unabhängig voneinander ausgewählt sind aus substituiertem oder unsubstituiertem Aryl, substituiertem oder unsubstituiertem C₆- bis C₂₄-Aryl, substituiertem oder unsubstituiertem Heteroaryl oder C₂- bis C₂₄-Heteroaryl, wobei die Substituenten an Ar¹, Ar² und Ar³ unabhängig voneinander ausgewählt sind aus einer elektronenziehenden Gruppe, NO₂, CN, Halogen, Cl, F, teilfluoriertes oder perfluoriertes Alkyl, teilfluoriertes oder perfluoriertes C₁- bis C₁₂-Alkyl, CF3, teilfluoriertes oder perfluoriertes Alkoxy, teilfluoriertes oder perfluoriertes C₁- bis C₆-Alkoxy oder D;
R', R'' und R‴ sind unabhängig voneinander ausgewählt aus substituiertem oder unsubstituiertem Aryl, substituiertem oder unsubstituiertem C₆- bis C₁₈-Aryl, substituiertem oder unsubstituiertem Heteroaryl, substituiertem oder unsubstituiertem C₂- bis C₁₈-Heteroaryl, elektronenziehende Gruppe, teilfluoriertem oder perfluoriertem Alkyl, teilweise fluoriertem oder perfluoriertem C₁- bis C₆-Alkyl, Halogen, F, CN und NO₂;
wobei in den Formeln (II) bis (IV) jedes Ar¹, Ar² und Ar³ mit dem entsprechenden R', R'' und R‴ einen substituierten oder unsubstituierten Carbocyclus oder vorzugsweise einen substituierten oder unsubstituierten Heterocyclus bilden kann, wobei die Substituenten eine elektronenziehende Gruppe, F, CN, perfluoriertes C₁- bis C₈-Alkyl, substituiertes oder unsubstituiertes C₆- bis C₁₈-Aryl, substituiertes oder unsubstituiertes C₂- bis C₁₈-Heteroaryl, substituiertes oder unsubstituiertes Carbocyclen, substituiertes oder unsubstituiertes Carbocycliden, substituiertes oder unsubstituiertes Alkylen, substituiertes oder unsubstituiertes Alkyliden sein können;
wobei die Verbindung der Formel (I) beim Messen mittels DSC während des Erhitzens mit 10 K/min keine Glasübergangstemperatur zeigt.

2. Verbindung nach Anspruch 1, wobei die Verbindung auf ≥ 150 °C erhitzt wurde.

3. Verbindung nach Anspruch
1 oder 2, mit einem LUMO-Energieniveau, ausgedrückt in der absoluten Skala, die sich auf ein Vakuumenergie-Niveau von ≤ -4,5 eV bezieht, berechnet unter Verwendung des hybriden Funktionals B3LYP mit einem Gaußschen 6-31G*- Basissatz, wie im Programmpaket TURBOMOLE V6.5 implementiert.

4. Verbindung nach einem der Ansprüche 1 bis 3, wobei die Verbindung eine Schmelztemperatur Tₘ von ≥200 °C aufweist.

5. Verbindung nach einem der Ansprüche 1 bis 4, wobei die Verbindung eine Schmelzenthalpie ΔHₘ von ≥ 30 kJ/mol aufweist.

6. Verbindung nach einem der Ansprüche 1 bis 5, wobei die Verbindung vier bis acht CN-Gruppen aufweist.

7. Verbindung nach einem der Ansprüche 1 bis 6, wobei die Verbindung eine Molekularmasse von ≥380 g/mol aufweist.

8. Verbindung nach einem der Ansprüche 1 bis 7, wobei mindestens eines der R', R'' und R‴ CN ist.

9. Verbindung nach einem der Ansprüche 1 bis 8, wobei mindestens eines der Elemente Ar¹, Ar² und Ar³ aus der Formel (V) ausgewählt ist: 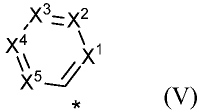
wobei X¹ ausgewählt ist aus CR¹ oder N;
X² ausgewählt ist aus CR² oder N;
X³ ausgewählt ist aus CR³ oder N;
X⁴ ausgewählt ist aus CR⁴ oder N;
X⁵ ausgewählt ist aus CR⁵ oder N;
wobei R¹, R², R³, R⁴, und R⁵ unabhängig voneinander ausgewählt sind aus einer elektronenziehenden Gruppe, NO₂, CN, Halogen, Cl, F, teilfluoriertem oder perfluoriertem Alkyl, teilfluoriertem oder perfluoriertem C₁- bis C₁₀-Alkyl, CF₃, teilfluoriertem oder perfluoriertem Alkoxy, teilfluoriertem oder perfluoriertem C₁- bis C₆-Alkoxy, D oder H;
wobei mindestens zwei von X¹ bis X⁵ unabhängig voneinander aus CR¹ bis CR⁵ ausgewählt sind; und
wobei das Sternchen "*" die Bindungsstelle kennzeichnet.

10. Organische Halbleiterschicht, welche mindestens eine Verbindung der Formel (I) nach einem der vorhergehenden Ansprüche 1 bis 9 aufweist.

11. Organische elektronische Vorrichtung, welche mindestens eine organische Halbleiterschicht nach Anspruch 10 aufweist.

12. Organische elektronische Vorrichtung nach Anspruch 11, wobei die organische elektronische Vorrichtung eine Anodenschicht, eine Kathodenschicht, mindestens eine photoaktive Schicht und mindestens eine organische Halbleiterschicht aufweist, wobei die mindestens eine organische Halbleiterschicht zwischen der Anodenschicht und der mindestens einen photoaktiven Schicht angeordnet ist.

13. Organische elektronische Vorrichtung nach einem der Ansprüche 11 bis 12, wobei die organische elektronische Vorrichtung mindestens zwei photoaktive Schichten aufweist, wobei mindestens eine organische Halbleiterschicht zwischen der ersten und der zweiten photoaktiven Schicht angeordnet ist.

14. Organische elektronische Vorrichtung nach einem der Ansprüche 11 bis 13, wobei die organische elektronische Vorrichtung eine lichtemittierende Vorrichtung, ein Dünnschichttransistor, eine Batterie, eine Anzeigevorrichtung oder eine Photovoltaikzelle ist, und vorzugsweise eine lichtemittierende Vorrichtung, vorzugsweise eine organische Leuchtdiode.

15. Anzeigevorrichtung, welche eine organische elektronische Vorrichtung nach einem der Ansprüche 11 bis 14 aufweist.

## Revendications

1. Un composé de formule (I) 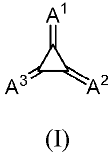
dans lequel A¹, A² et A³ sont représentés par la formule (II), (III), (IV), respectivement 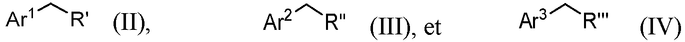
dans lequel Ar¹, Ar² et Ar³ sont sélectionnés indépendamment parmi un aryle substitué ou non substitué, un aryle C₆ à C₂₄ substitué ou non substitué, un hétéroaryle substitué ou non substitué ou un hétéroaryle C₂ à C₂₄, dans lequel les substituants sur Ar¹, Ar² et Ar³ sont sélectionnés indépendamment parmi un groupe attracteur d'électrons, NO₂, CN, un halogène, Cl, F, un alkyle partiellement fluoré ou perfluoré, un alkyle C₁ à C₁₂ partiellement fluoré ou perfluoré, CF3, un alcoxy partiellement fluoré ou perfluoré, un alcoxy C₁ à C₆ partiellement fluoré ou perfluoré ou D;
R', R" et R‴ sont sélectionnés indépendamment parmi un aryle substitué ou non substitué, un aryle C₆ à C₁₈ substitué ou non substitué, un hétéroaryle substitué ou non substitué, un hétéroaryle C₂ à C₁₈ substitué ou non substitué, un groupe attracteur d'électrons, un alkyle partiellement fluoré ou perfluoré, un alkyle C₁ à C₆ partiellement fluoré ou perfluoré, un halogène, F, CN, et NO₂;
dans lequel dans les formules (II) à (IV), chaque Ar¹, Ar² et Ar³ avec les R', R" et R‴ correspondants peuvent former un carbocyle substitué ou non substitué ou, de préférence, un hétérocycle substitué ou non substitué, dans lequel les substituants peuvent être un groupe attracteur d'électrons, F, CN, un alkyle C₁ à C₈ perfluoré, un aryle C₆ à C₁₈ substitué ou non substitué, un hétéroaryle C₂ à C₁₈ substitué ou non substitué, un carbocyclène substitué ou non substitué, un carbocyclidene substitué ou non substitué, un alkylène substitué ou non substitué, un alkylidène substitué ou non substitué;
dans lequel le composé de formule (I) ne présente pas de température de transition vitreuse lorsqu'il est mesuré par DSC pendant le chauffage à 10 K/min.

2. Composé selon la revendication 1, dans lequel le composé a été chauffé à ≥ 150 °C.

3. Composé selon la revendication
1 ou 2, ayant un niveau d'énergie LUMO, estimé selon
l'échelle absolue relative au niveau d'énergie du vide étant zéro, de ≤ -4,5 eV, calculé en utilisant la fonctionnelle hybride B3LYP avec un ensemble de base gaussien 6-31G* comme appliqués dans le progiciel TURBOMOLE V6.5.

4. Composé selon l'une quelconque des revendications 1 à 3, dans lequel le composé possède une température de fusion Tₘ de ≥200 °C.

5. Composé selon l'une quelconque des revendications 1 à 4, dans lequel le composé possède une enthalpie de fusion ΔHₘ de ≥ 30 kJ/mol.

6. Composé selon l'une quelconque des revendications 1 à 5, dans lequel le composé possède quatre à huit groupes CN.

7. Composé selon l'une quelconque des revendications 1 à 6, dans lequel le composé possède une masse moléculaire de ≥380 g/mol.

8. Composé selon l'une quelconque des revendications 1 à 7, selon lequel au moins l'un des R', R" et R‴ est CN.

9. Composé selon l'une quelconque des revendications 1 à 8, dans lequel au moins l'un des Ar¹, Ar² et Ar³ est choisi dans la formule (V): 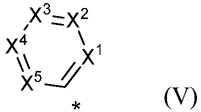
dans lequel X¹ est sélectionné parmi CR¹ ou N;
X² est sélectionné parmi CR² ou N;
X³ est sélectionné parmi CR³ ou N;
X⁴ est sélectionné parmi CR⁴ ou N;
X⁵ est sélectionné parmi CR⁵ ou N;
dans lequel R¹, R², R³, R⁴, et R⁵ sont sélectionnés indépendamment parmi un groupe attracteur d'électrons, NO₂, CN, un halogène, Cl, F, un alkyle partiellement fluoré ou perfluoré, un alkyle C₁ à C₁₀ partiellement fluoré ou perfluoré, CF₃, un alcoxy partiellement fluoré ou perfluoré, un alcoxy C₁ à C₆ partiellement fluoré ou perfluoré, D ou H;
dans lequel au moins deux des X¹ à X⁵ sont sélectionnés indépendamment parmi CR¹ à CR⁵; et
dans lequel l'astérisque "*" désigne la position de liaison.

10. Couche semi-conductrice organique comprenant au moins un composé de formule (I) selon l'une quelconque des revendications précédentes 1 à 9.

11. Dispositif électronique organique comprenant au moins une couche semi-conductrice organique selon la revendication 10.

12. Dispositif électronique organique selon la revendication 11, dans lequel le dispositif électronique organique comprend une couche d'anode, une couche de cathode, au moins une couche photoactive et au moins une couche semi-conductrice organique, dans lequel la ou les couches semi-conductrices organiques sont disposées entre la couche d'anode et au moins une couche photoactive.

13. Dispositif électronique organique selon l'une quelconque des revendications 11 à 12, dans lequel le dispositif électronique organique comprend au moins deux couches photoactives, dans lequel au moins une couche semi-conductrice organique est disposée entre la première et la deuxième couche photoactive.

14. Dispositif électronique organique selon l'une quelconque des revendications 11 à 13, dans lequel le dispositif électronique organique est un dispositif électroluminescent, un transistor à couches minces, une batterie, un dispositif d'affichage ou une cellule photovoltaïque, et de préférence un dispositif électroluminescent, de préférence une diode électroluminescente organique.

15. Dispositif d'affichage comprenant un dispositif électronique organique selon l'une quelconque des revendications 11 à 14.
